# EUROPEAN PATENT APPLICATION

(11) **EP 4 722 232 A1**
(43) Date of publication of application: **08.04.2026**
(21) Application number: 24814629.2
(22) Date of filing: 31.05.2024
(51) Int. Cl.: C07K 14/32, C07K 14/33

(54) **SINGLE-CHAIN POLYPEPTIDE ACTIVATED IN USE**

(30) Priority: 31.05.2023 CN 202310640693
(71) Applicant: YSTE (Hainan) Aesthetic Medicine Health Technology Co., Ltd., Sanya, Hainan 572025 (CN); Kangma-Healthcode (Shanghai) Biotech Co., Ltd, Pudong New Area Shanghai 201321 (CN)
(72) Inventor: GUO, Min, Shanghai 201321 (CN); YANG, Longjin, Shanghai 201321 (CN); XIAO, Song, Shanghai 201321 (CN); HU, Jiabao, Shanghai 201321 (CN); LIU, Zhang, Shanghai 201321 (CN); YU, Xue, Shanghai 201321 (CN)
(74) Representative: Dai, Simin
(86) International application number: PCT/CN2024/096882
(87) International publication number: WO 2024/245435

(57) **Abstract**

Provided are a single-chain polypeptide which is highly activated after entering a specific use site, attenuated and can be safer and more convenient to produce, and the corresponding use thereof, a nucleic acid encoding same and a preparation method therefor. The single-chain polypeptide comprises: a first domain, an intermediate amino acid sequence region, and a second domain, wherein the intermediate amino acid sequence region is located between the first domain and the second domain, and contains at least one first enzyme cleavage site, the first enzyme cleavage site is designed to be capable of being highly specifically cleaved by a first protease present in a specific part of the human body, and the single-chain polypeptide is converted into an activated form capable of inhibiting the release of an information substance after being cleaved by the first protease at the first enzyme cleavage site.

## Description

### TECHNICAL FIELD

The present disclosure belongs to the field of biotechnology, specifically relates to a single-chain polypeptide activated in use, and specifically relates to a single-chain polypeptide, as well as the corresponding use thereof, a nucleic acid encoding the same, and a preparation method therefor.

### BACKGROUND

At present, applications can be achieved through some toxin molecules with specific cleavage components, for example:
(1) Therapeutic applications: treatment of diseases such as hemifacial spasm, idiopathic blepharospasm, spasmodic torticollis, spastic cerebral palsy, post-stroke limb spasticity, tremor, and other various types of dystonia;
(2) Applications in the field of minimally invasive plastic surgery:
   including removal of various types of dynamic wrinkles such as glabellar lines, forehead lines, and crow's feet; facial contour enhancement such as adjustment of eyebrow height, mentalis muscle relaxation, jawline lifting, and platysmal band injection; body contouring such as masseter muscle reduction, gastrocnemius muscle reduction, and trapezius muscle reduction.

However, there is currently no product that can be safely and effectively produced and safely and effectively used to achieve the aforementioned effects.

### SUMMARY

The present disclosure provides a single-chain polypeptide that is highly activated upon reaching a specific site of use, attenuated, and capable of safer and more convenient production, as well as the corresponding use thereof, a nucleic acid encoding the same, and a preparation method therefor.

For this purpose, the present disclosure provides the following technical solutions:
The present disclosure provides a single-chain polypeptide, characterized by comprising: a first domain, an intermediate amino acid sequence region, and a second domain, wherein the intermediate amino acid sequence region is located between the first domain and the second domain, and contains at least one first enzyme cleavage site; the first enzyme cleavage site is designed to be capable of being highly specifically cleaved by a first protease present at a specific site of the human body, and the single-chain polypeptide is converted into an activated form capable of inhibiting the release of a signaling substance upon cleavage of the first enzyme cleavage site by the first protease.

The single-chain polypeptide provided by the present disclosure is further characterized in that the first domain has a molecular weight of approximately 50 kDa, the second domain has a molecular weight of approximately 100 kD, and the first domain possesses cleavage activity.

The single-chain polypeptide provided by the present disclosure is further characterized in that the signaling substance is an intercellular signaling substance, preferably any one of a neurotransmitter, an endocrine hormone, a local chemical mediator, and a gaseous signaling molecule; more preferably, the signaling substance is acetylcholine.

The single-chain polypeptide provided by the present disclosure is further characterized in that the first domain is a non-toxic cellular protease or a fragment thereof capable of cleaving an exocytosis fusion protein of a target cell; the second domain comprises a binding moiety and a transporting moiety, wherein the binding moiety is capable of binding to a receptor of the target cell and entering an endosome of the target cell via receptor-mediated endocytosis, and the transporting moiety is capable of transporting the first domain into a cytosol of the target cell;
preferably, the first enzyme cleavage site is located between the first domain and the transporting moiety; and/or the binding moiety is located at the carboxyl terminus with a molecular weight of approximately 50 kD, and the transporting moiety is located at the amino terminus with a molecular weight of approximately 50 kD;
another preferably, the exocytosis fusion protein is a neuronal SNARE protein, and/or the target binding site is a receptor on the presynaptic membrane of a neuron.

The single-chain polypeptide provided by the present disclosure is further characterized in that the first domain comprises at least a portion of a *Clostridium botulinum* neurotoxin L chain;
preferably, the portion of the *Clostridium botulinum* neurotoxin L chain is a neurotoxin L chain of one of *Clostridium botulinum* subtypes A, B, C, D, and E or is derived therefrom; and/or the first domain comprises an amino acid sequence having at least 35%, 50%, 60%, 70%, 75%, 80%, 85%, 90%, 99%, or 100% identity to SEQ ID NO: 10, or comprises an amino acid sequence having at least 35%, 50%, 60%, 70%, 75%, 80%, 85%, 90%, 99%, or 100% identity to any one of SEQ ID NOs: 11 to 16 with removal of a naturally occurring cleavage sequence; and/or
the second domain comprises at least a portion of a *Clostridium botulinum* neurotoxin H chain; preferably, the portion of the *Clostridium botulinum* neurotoxin H chain is a neurotoxin H chain of one of *Clostridium botulinum* (*C. Botulinum)* subtype A, *Clostridium botulinum* subtype B, *Clostridium botulinum* subtype C, *Clostridium botulinum* subtype D, *Clostridium botulinum* subtype E, *Clostridium botulinum* subtype F, *Clostridium botulinum* subtype G, *Clostridium baratii* (*C. Baratii*), and *Clostridium butyricum* (*C. Butyricum*), or is derived therefrom; or the portion of the *Clostridium botulinum* neurotoxin H chain is a neurotoxin H chain of *Clostridium tetani* (*C. Tetani*) or is derived therefrom; and/or
the second domain comprises an amino acid sequence having at least 35%, 50%, 60%, 70%, 75%, 80%, 85%, 90%, 99%, or 100% identity to any one of SEQ ID NOs: 17 to 23.

The single-chain polypeptide provided by the present disclosure is further characterized in that the number of amino acids in the linker (L1) between the first domain and the intermediate amino acid sequence region is 0 to 20, 0 to 10, or 0 to 5; and/or the number of amino acids in the linker (L2) between the intermediate amino acid sequence region and the second domain is 0 to 20, 0 to 10, or 0 to 5.

The single-chain polypeptide provided by the present disclosure is further characterized in that the first protease is not a protease present in the human digestive tract;
preferably, the specific site of the human body is human skin tissue, and/or at least one of the first proteases is selected from the group consisting of:
an epidermal-specific kallikrein-related peptidase (KLK) (*e.g.,* KLK5, KLK6, KLK7), a SASPase protease, a Caspase protease (e.g., Caspase-14), a Furin protease, and any one or more of their respective variants retaining proteolytic activity; and/or
the first enzyme cleavage site comprises an amino acid sequence having at least 50%, 60%, 85%, 90%, 99%, or 100% identity to any one of SEQ ID NOs: 1 to 4, SEQ ID NO: 50, and SEQ ID NO: 51.

In SEQ ID NO: 50 and SEQ ID NO: 51, "X" in RXXR and RRXX is any amino acid; preferably, "XX" in RXXR is RK, TK, or TR; "XX" in RRXX is SV or KR.

The single-chain polypeptide provided by the present disclosure is further characterized by comprising any one or more of a signal peptide for increased expression, a quantitative protein for quantification, and a tag protein; further, one or more of the signal peptide, the quantitative protein, and the tag protein are respectively defined as follows: the signal peptide comprises an amino acid sequence having at least 80%, 85%, 90%, 99%, or 100% identity to SEQ ID NO: 24; the quantitative protein is a fluorescent protein; the tag protein is one or more of a His tag, a GST tag, an MBP tag, and a Strep-tagII tag.

The single-chain polypeptide provided by the present disclosure is further characterized by comprising two second enzyme cleavage sites located at the N-terminus of the first domain and the C-terminus of the second domain, respectively, and a second protease that cleaves the second enzyme cleavage site is incapable of cleaving the first enzyme cleavage site.

The single-chain polypeptide provided by the present disclosure is further characterized in that the first enzyme cleavage site and/or the second enzyme cleavage site are typically not cleaved by proteases encountered during expression of the single-chain polypeptide within a host cell or during synthesis of the single-chain polypeptide in an *in vitro* synthesis system involving a cell extract derived from the host cell.

The single-chain polypeptide provided by the present disclosure is further characterized in that the second enzyme cleavage site is designed to be highly specific for the second protease; preferably, the second protease is selected from a non-human enterokinase, a tobacco etch virus protease, a protease derived from *Bacillus subtilis,* a protease derived from *Bacillus amyloliquefaciens,* a protease derived from rhinovirus, papain, a homolog of papain of an insect, or a homolog of papain of a crustacean; and/or
the second enzyme cleavage site comprises an amino acid sequence having at least 85%, 90%, 99%, or 100% identity to any one of SEQ ID NOs: 5 to 9.

The single-chain polypeptide provided by the present disclosure is further characterized in that the intermediate amino acid sequence region further comprises XTXS, wherein the X is an amino acid other than K; preferably, the X is Q; and/or
the structure of the intermediate amino acid sequence region from the N-terminus to the C-terminus is:
XTXS-linker (L3)-at least one first enzyme cleavage site; further, the number of amino acids in L3 is 0 to 20, 0 to 10, or 0 to 5; further, there is one first enzyme cleavage site.

The single-chain polypeptide provided by the present disclosure is further characterized in that when the second domain comprises an amino acid sequence having at least 80%, 85%, 90%, 95%, 99%, or 100% identity to SEQ ID NO: 17, the lysine at the position corresponding to position 423 of SEQ ID NO: 17 in the amino acid sequence of the second domain is mutated to a non-lysine residue.

The single-chain polypeptide provided by the present disclosure is further characterized in that the first domain, the intermediate amino acid sequence region, and the second domain are connected from the N-terminus to the C-terminus; and/or when there are multiple first enzyme cleavage sites, the multiple first enzyme cleavage sites are sequentially connected from the N-terminus to the C-terminus, wherein all of the first enzyme cleavage sites are identical or different from one another, and the number of amino acids in the linker (L4) between the multiple enzyme cleavage sites is 0 to 20, 0 to 10, or 0 to 5.

The present disclosure further provides a use of the single-chain polypeptide described above, wherein the use comprises any one or more of the following: skin care, plastic surgery, body contouring, and therapy by inducing muscle relaxation or relief of muscle spasm.

The present disclosure further provides a use of the single-chain polypeptide described above in the manufacture of a product or medicament for a specific purpose, wherein the specific purpose comprises any one or more of the following: skin care, plastic surgery, body contouring, and therapy by inducing muscle relaxation or relief of muscle spasm.

The present disclosure further provides a product or medicament of a single-chain polypeptide, wherein the product or medicament comprises the single-chain polypeptide described above; preferably, the product or medicament has a use comprising any one or more of the following: skin care, plastic surgery, body contouring, and therapy by inducing muscle relaxation or relief of muscle spasm;
more preferably, the product or medicament is applied to a specific site of the human body by means of topical application, spraying, external application, or injection.

The present disclosure further provides a nucleic acid, wherein the nucleic acid comprises a nucleotide sequence encoding the single-chain polypeptide described above.

The present disclosure further provides a vector, wherein the vector comprises the nucleic acid described above.

The present disclosure further provides a host cell, integrated with the nucleic acid described above and/or comprising the vector described above.

The host cell provided by the present disclosure is further characterized in that the host cell is derived from a prokaryotic cell or a eukaryotic cell; further, the cell is selected from the following groups: one or more of an *Escherichia coli* cell, a human-derived cell, a Chinese hamster ovary cell, an insect cell, a wheat germ cell, a rabbit reticulocyte, and a yeast cell, or a combination thereof.

The host cell provided by the present disclosure is further characterized in that the host cell is derived from a yeast cell; further, the yeast cell is selected from one or more of *Saccharomyces cerevisiae and Kluyveromyces,* or a combination thereof; in another preferred example, the *Kluyveromyces* is selected from one or more of *Kluyveromyces lactis, Kluyveromyces marxianus,* and *Kluyveromyces dobzhanskii,* or a combination thereof.

The present disclosure further provides an *in vitro cell-free* synthesis system, wherein an mRNA or DNA encoding the single-chain polypeptide described above is used as a template to synthesize the single-chain polypeptide.

The *in vitro* cell-free synthesis system provided by the present disclosure is further characterized by comprising any one or more of the following:
(1) an mRNA or DNA template encoding the single-chain polypeptide;
(2) a cell extract, wherein the cell extract is a yeast cell extract; further, the cell extract is derived from a strain selected from the group consisting of *Saccharomyces cerevisiae, Pichia pastoris,* and *Kluyveromyces,* or a combination thereof; preferably, the yeast cell extract is derived from *Kluyveromyces,* more preferably *Kluyveromyces lactis;*
(3) any one or more of the following components:
   an amino acid mixture, dNTP, RNA polymerase, DNA polymerase, an energy supply system, polyethylene glycol, and an aqueous solvent.

The present disclosure also provides a preparation method for the single-chain polypeptide described above, wherein
an mRNA or DNA encoding the single-chain polypeptide described above is used as a template to perform an *in vitro* synthesis reaction to obtain the single-chain polypeptide;
preferably, the *in vitro* synthesis reaction is performed in a reaction system comprising the *in vitro* cell-free synthesis system described above;
further, the DNA template and other components involved in the *in vitro* synthesis reaction have a volume ratio of 1:10 to 1:50, or 1:20 to 1:40, or 1:25 to 1:35, or 1:30.

The preparation method provided by the present disclosure is further characterized in that the *in vitro* cell-free synthesis system comprises a yeast cell extract, an amino acid mixture, dNTP, RNA polymerase, DNA polymerase, an energy supply system, polyethylene glycol, and an aqueous solvent;
further:
the yeast cell extract is derived from a strain selected from the group consisting of *Saccharomyces cerevisiae, Pichia pastoris,* and *Kluyveromyces,* or a combination thereof; preferably, the yeast cell extract is derived from *Kluyveromyces,* more preferably *Kluyveromyces lactis.*

The preparation method provided by the present disclosure is further characterized in that the yeast cell extract accounts for 50 to 80% of the total volume of the *in vitro* cell-free protein synthesis system.

The preparation method provided by the present disclosure is further characterized by comprising: digesting the obtained single-chain polypeptide comprising the second enzyme cleavage site with the second protease to obtain a single-chain polypeptide comprising only the first domain, the intermediate amino acid sequence region, and the second domain.

The present disclosure further provides a use of the nucleic acid described above, the vector described above, the host cell described above, or the *in vitro* cell-free synthesis system described above in the manufacture of the single-chain polypeptide described above.

### Effects and advantages

The present disclosure provides a single-chain polypeptide activated in use, and specifically relates to a single-chain polypeptide, as well as the corresponding use thereof, a nucleic acid encoding the same, and a preparation method therefor, having at least the following advantages:
(1) The single-chain polypeptide provided by the present disclosure comprises a first enzyme cleavage site between the first domain and the second domain, which is designed to be highly specifically cleaved by a first protease present at a specific site of the human body. Thus, when applied to a specific site, the first enzyme cleavage site is highly specifically cleaved by the first protease present at the specific site of the human body upon reaching the specific site, thereby converting the single-chain polypeptide into an activated form. Compared with the currently available pre-activated forms, the safety of use is greatly improved, especially when applied to skin tissue. The safety profile allows for safe and convenient administration through methods such as topical application, thereby improving the usability, practicality, and universality of the product.
(2) Further, since the first protease is not present in the human digestive tract, accidental ingestion will not lead to activation, further improving safety of use.
(3) Moreover, since the first enzyme cleavage site of the single-chain polypeptide is designed to be highly specifically cleaved by the first protease present at the specific site of the human body, and the single-chain polypeptide is only highly converted into the activated form after such cleavage, the production process becomes safer, which not only ensures the safety of upstream production but also ensures the safety of downstream production. Particularly, when the single-chain polypeptide of the present disclosure is synthesized *in vitro* using an mRNA or DNA encoding the single-chain polypeptide of the present disclosure as a template, there are no risks associated with strain leakage in intracellular expression systems. Additionally, the process has a short cycle, simple methodology, and low cost, further improving safety and practicality.

### BRIEF DESCRIPTION OF THE DRAWINGS

FIG. 1 is an electrophoretogram of the modified protein obtained after elution and purification at pH 8.0 in the example;
FIG. 2 is a reference diagram for efficacy scoring in animal experiments;
FIG. 3 shows the results of the animal experiment in Example 7;
FIG. 4 and FIG. 5 show the substrate conversion rates for different concentrations of substrates CFP-SNAP25(17aa)-YFP and CFP-SNAP25(66aa)-YFP in Example 6, respectively;
FIG. 6 shows the comparative results of the activity effects between TEV single digestion products and double digestion products in Example 6 to exclude any effect from TEV protease;
FIG. 7 shows the comparative results of the activity effects between double digestion products and KLK5 protease in Example 6 to exclude any effect from KLK5 protease;
FIG. 8 shows the *in vitro* activity assay of single cleavage at multiple first enzyme cleavage sites in Example 6;
FIG. 9 shows the LD50 test results in Example 8;
FIG. 10 shows the ED50 test results in Example 9.

### DETAILED DESCRIPTION OF THE PREFERRED EMBODIMENT

The specific embodiments of the present disclosure are described below with reference to the accompanying drawings. For the specific methods or materials used in the examples, those skilled in the art may make conventional replacement selections based on the technical ideas of the present disclosure and according to existing technologies, and are not limited to the specific descriptions in the examples of the present disclosure.

Unless otherwise specified, the methods used in the examples are conventional methods. Unless otherwise specified, the materials and reagents used are commercially available.

### 1. Description of single-chain polypeptides

Signaling transduction refers to the process of transmitting extracellular information into the cell. It is a fundamental concept in cell communication, emphasizing the generation, secretion, and transmission of signals, *i.e.,* the release of signaling substances from synthesizing cells, followed by transmission. After a receptor recognizes and binds to the corresponding ligand (first messenger), intracellular substances such as cyclic adenosine monophosphate (cAMP), cyclic guanosine monophosphate (cGMP), calcium ions, and inositol phospholipids (second messengers) increase in level. These substances are involved in various biological regulatory processes within the cell, whereby the acquired information is amplified, diversified, integrated, and transmitted to effectors to exert specific physiological functions or pharmacological effects.

Signaling transduction between human cells can be achieved through direct contact between adjacent cells, but more importantly and more commonly, it is achieved through the secretion of various chemical substances by cells to regulate their own metabolism and functions, as well as those of other cells. Therefore, in the human body, signaling transduction pathways typically consist of specific cells that secrete and release signaling substances, signaling substances (including intercellular and intracellular signaling substances, carriers, transport pathways, *etc.*)*,* and target cells (including specific receptors, *etc.*)*.*

Signaling substances can be categorized into intercellular signaling substances and intracellular signaling molecules.

Chemical substances secreted by cells that regulate the life activities of target cells are collectively referred to as intercellular signaling substances, also known as first messengers. Depending on the mode of secretion, intercellular signaling substances can be further categorized into neurotransmitters, endocrine hormones, local chemical mediators, and gaseous signaling molecules. Chemical substances that mediate the transmission of regulatory signals within cells are referred to as intracellular signaling substances, which exhibit diverse compositions. Small molecule compounds that transmit signals within cells, such as Ca²⁺, cAMP, cGMP, DAG, IP3, Cer, arachidonic acid, and their metabolites, are typically referred to as second messengers. Substances responsible for transmitting signals between the nucleus and the cytoplasm are referred to as third messengers, which can bind to specific sequences of target genes and function as transcription factors or transcriptional regulators.

Neurotransmitters are signaling substances that transmit signals between neurons or between neurons and effector cells, such as muscle cells and gland cells. Based on their chemical composition, neurotransmitters are mainly categorized into cholines (*e.g.,* acetylcholine, abbreviated as ACh), monoamines (e.g., norepinephrine, dopamine, and serotonin), amino acids (excitatory transmitters such as glutamate and aspartate; inhibitory transmitters such as γ-aminobutyric acid, glycine, and taurine), and neuropeptides.

The synthesis of acetylcholine (ACh) primarily occurs at cholinergic nerve terminals. When a nerve impulse reaches the nerve terminal, the vesicle membrane fuses with the presynaptic membrane, releasing acetylcholine into the synaptic cleft, which then activates corresponding receptors on the postsynaptic membrane to induce a series of physiological effects. Simultaneously, acetylcholine is inactivated by hydrolysis into choline and acetate by cholinesterase (ChE) at the nerve terminal. A portion of the choline is reabsorbed by the cholinergic nerve terminal and reused for the synthesis of new acetylcholine.

Existing studies have demonstrated that inhibition of acetylcholine transmission can lead to muscle relaxation, relief of muscle spasm, and modulation of hypertonia and dyskinesia as well as dynamic wrinkles. Additionally, it can reduce muscle volume by inducing muscle disuse atrophy, thereby enabling body contouring.

Non-cytotoxic proteases function through proteolytic cleavage of intracellular transport proteins known as SNARE proteins (see Gerald K (2002), Cell and Molecular Biology (4th Edition) John Wiley & Sons, Inc.).

The acronym SNARE is derived from the term Soluble NSF Attachment Receptor, wherein NSF stands for N-ethylmaleimide-Sensitive Factor.

SNARE proteins are essential for intracellular vesicle fusion and thus necessary for vesicle-mediated transport of secretory molecules. The function of non-cytotoxic proteases is mediated by zinc-dependent endopeptidase activity, exhibiting high substrate specificity for SNARE proteins. SNARE proteins are associated with the membrane of a secretory vesicle or the cell membrane and facilitate exocytosis of molecules by mediating the fusion of the secretory vesicle with the cell membrane, thereby allowing the release of vesicular contents into the extracellular space. Cleavage of neuronal SNAREs prevents the release of neurotransmitters, leading to paralysis. Therefore, once non-cytotoxic proteases are delivered to the desired target cells, they are capable of inhibiting secretion by the target cells.

Three proteins located at the synaptic terminal, namely synaptobrevin (VAMP) on the synaptic vesicle membrane, syntaxin on the presynaptic membrane, and SNAP-25, are all referred to as SNARE proteins.

The single-chain polypeptide provided by the present disclosure comprises: a first domain, an intermediate amino acid sequence region, and a second domain.

There may be intervening amino acids (linker, L1 > 0) between the intermediate amino acid sequence region and the first domain, or they may be directly connected (L1 = 0). Similarly, there may be intervening amino acids (linker, L2 > 0) between the first cleavage site and the second domain, or they may be directly connected (L2 = 0).

In one example, the number of amino acids in the linker (L1) between the first domain and the intermediate amino acid sequence region is 0 to 20, 0 to 10, or 0 to 5.

In one example, the number of amino acids in the linker (L2) between the intermediate amino acid sequence region and the second domain is 0 to 20, 0 to 10, or 0 to 5.

The intermediate amino acid sequence region contains at least one first enzyme cleavage site. When there are multiple first enzyme cleavage sites:
these first enzyme cleavage sites may be identical, or all of them are identical or different from one another;
the multiple first enzyme cleavage sites are sequentially connected from the N-terminus to the C-terminus;
the number of amino acids in the linker (L4) between the multiple enzyme cleavage sites is 0 to 20, 0 to 10, or 0 to 5.

The first enzyme cleavage site is designed to be capable of being highly specifically cleaved by a first protease present at a specific site of the human body. The single-chain polypeptide is converted into an activated form upon cleavage of the first enzyme cleavage site by the first protease, and the activated single-chain polypeptide can inhibit the release of the signaling substance described above.

The specific site of the human body refers to the site within the human body where the single-chain polypeptide is delivered during use, such as the digestive tract, skin tissue, or cells. During use, the method of delivery to the specific site of the human body may vary; for example, in the case of skin tissue, it may be delivered by means of topical application, external application, spraying, injection, *etc.*

As used herein, the phrase "being highly specifically cleaved" means that, under normal conditions, an enzyme cleavage site is primarily recognized and cleaved by a corresponding specific protease and cannot be cleaved by other proteases. In other words, under most conditions, the enzyme cleavage site has a one-to-one correspondence with its corresponding protease.

Specifically, the phrase "the first enzyme cleavage site is designed to be capable of being highly specifically cleaved by a first protease present at a specific site of the human body where the single-chain polypeptide is applied" means that, under most conditions, the first enzyme cleavage site has a one-to-one correspondence with one or several first proteases present at the specific site of the human body. For example, when the site of use is the digestive tract, the first enzyme cleavage site has a one-to-one correspondence with one or several proteases present in the digestive tract. For another example, when the site of use is skin tissue, the first enzyme cleavage site has a one-to-one correspondence with one or several proteases present in the skin tissue.

Accordingly, the single-chain polypeptide is only activated when applied at a specific site of the human body where the first protease described above is present. For example, when the single-chain polypeptide is applied to the skin surface and penetrates into the skin tissue, the first enzyme cleavage site is cleaved by the highly specific first protease present in the skin tissue, thereby converting the single-chain polypeptide into an activated form capable of inhibiting the release of signaling substances.

The activated form of the single-chain polypeptide is a double-chain structure in which the first domain and the second domain are connected via a disulfide bond. Upon activation, the disulfide bond is reduced, thereby generating a product with cleavage activity capable of inhibiting the release of signaling substances such as neurotransmitters. As used herein, cleavage activity refers to the cleavage of proteins associated with vesicles and/or the plasma membrane that are involved in exocytosis of cells:
essentially, the first domain and the second domain are separated by the intermediate amino acid sequence region, which contains the first enzyme cleavage site but does not contain a non-wild-type cleavable amino acid sequence, *i.e.,* a cleavable amino acid sequence not naturally occurring between the first and second domains (the wild-type or naturally occurring cleavable amino acid sequence refers to a sequence that is naturally occurring in the single-chain polypeptide and whose cleavage enables conversion of the first and second domains from a single-chain structure to an active double-chain structure). This is equivalent to replacing the function of the wild-type cleavable amino acid sequence with the first enzyme cleavage site, ensuring that the cleavage occurs only under the highly specific action of the first protease. For example, when the first domain is the light chain of botulinum toxin type A and the second domain is the light chain of botulinum toxin type A, the intermediate amino acid sequence region does not contain the naturally cleavable sequence "KTKSLDKGYNK" (a natural cleavage site sequence).

In one example, the signaling substance is acetylcholine.

Experimental results have demonstrated that the activated form of the single-chain polypeptide, namely the double-chain structure described above, can exert effects such as muscle relaxation, meaning that it possesses the function of inhibiting the transmission of signaling substances such as neurotransmitters.

Preferably, the first domain, the first enzyme cleavage site, and the second domain are connected from the N-terminus to the C-terminus.

In one example, the first domain has a molecular weight of approximately 50 kDa, and the second domain has a molecular weight of approximately 100 kD.

In a specific example, in the single-chain polypeptide designed in the present disclosure, the first domain is a non-toxic cellular protease or a fragment thereof capable of cleaving an exocytosis fusion protein of a target cell. In one example, the cell is a neuronal cell, the exocytosis fusion protein is a neuronal SNARE protein, and/or the target binding site is a receptor on the presynaptic membrane of a neuron.

When the exocytosis fusion protein is a neuronal SNARE protein, cleavage of the SNARE protein, e.g., prevents the release of neurotransmitters such as acetylcholine, thereby blocking the physiological function of cholinergic neurotransmission.

In one example, the SNARE protein is selected from VAMP, syntaxin, and SNAP-25.

The second domain comprises a binding moiety and a transporting moiety.

The binding moiety is capable of binding to a receptor of the target cell and entering an endosome of the target cell via receptor-mediated endocytosis, e.g., binding to an ectoacceptor on the presynaptic membrane of nerve terminals.

The transporting moiety is capable of transporting the first domain into a cytosol of the target cell.

Through binding of the binding moiety of the second domain to the receptor of the target cell and internalization into the endosome of the target cell via receptor-mediated endocytosis, the transporting moiety transports the first domain into the cytosol of the target cell. In the cytosol, the second domain is cleaved via reduction of the disulfide bond, releasing the first domain into the cytosol, thereby cleaving SNARE proteins and inhibiting the release of neurotransmitters, which leads to muscle relaxation, relief of muscle spasm, and subsequent modulation of hypertonia and dyskinesia as well as dynamic wrinkles. Additionally, it can reduce muscle volume by inducing muscle disuse atrophy, thereby enabling body contouring.

The binding moiety is located at the carboxyl terminus with a molecular weight of approximately 50 kD, and the transporting moiety is located at the amino terminus with a molecular weight of approximately 50 kD.

In one example, the first domain comprises at least a portion of a *Clostridium botulinum* neurotoxin L chain; preferably, the portion of the *Clostridium botulinum* neurotoxin L chain is a neurotoxin L chain of one of *Clostridium botulinum* subtypes A, B, C, D, and E or is derived therefrom; and/or
the first domain comprises an amino acid sequence having at least 35%, 50%, 60%, 70%, 75%, 80%, 85%, 90%, 99%, or 100% identity to SEQ ID NO: 10, or comprises an amino acid sequence having at least 35%, 50%, 60%, 70%, 75%, 80%, 85%, 90%, 99%, or 100% identity to any one of SEQ ID NOs: 11 to 16 with removal of a naturally occurring cleavage sequence.

In one example, the second domain comprises at least a portion of a *Clostridium botulinum* neurotoxin H chain;
preferably, the portion of the *Clostridium botulinum* neurotoxin H chain is a neurotoxin H chain of one of *Clostridium botulinum* (*C. Botulinum)* subtype A, *Clostridium botulinum* subtype B, *Clostridium botulinum* subtype C, *Clostridium botulinum* subtype D, *Clostridium botulinum* subtype E, *Clostridium botulinum* subtype F, *Clostridium botulinum* subtype G, *Clostridium baratii (C. Baratii*), and *Clostridium butyricum* (*C. Butyricum*), or is derived therefrom; or the portion of the *Clostridium botulinum* neurotoxin H chain is a neurotoxin H chain of *Clostridium tetani (C. Tetani*) or is derived therefrom; and/or
the second domain comprises an amino acid sequence having at least 35%, 50%, 60%, 70%, 75%, 80%, 85%, 90%, 99%, or 100% identity to any one of SEQ ID NOs: 17 to 23.

Further, the first protease is not a protease present in the human digestive tract, meaning that there is no protease capable of cleaving the first enzyme cleavage site with high specificity in the human digestive tract. In other words, under most normal conditions, the first enzyme cleavage site cannot be cleaved by a protease present in the human digestive tract, thereby avoiding toxicity issues caused by accidental ingestion into the digestive tract during use and ensuring safety of use.

As used herein, the digestive tract refers to the long muscular canal extending from the oral cavity to the pharynx, esophagus, stomach, small intestine, large intestine, and terminating at the anal canal, including the oral cavity, pharynx, esophagus, stomach, small intestine (duodenum, jejunum, ileum), large intestine (cecum, appendix, colon, rectum), and anal canal.

In one example, the specific site of the human body is the skin tissue, meaning that the first protease is present in human skin tissue.

As used herein, the term "skin tissue" refers to the entire "skin" of the body, including the scalp and mucosa. The epidermis is the outermost layer of the skin, covering the entire body and serving a protective function.

The skin tissue can be divided into three main layers: the epidermis, dermis, and subcutaneous tissue.

### (I) Epidermis

The epidermis is the outermost layer of the skin, covering the entire body and serving a protective function. The epidermis lacks blood vessels (thus does not bleed when scratched) but contains numerous fine nerve endings. Based on cell morphology, the epidermis can be divided into five layers, from outermost to innermost: the stratum corneum, stratum lucidum, stratum granulosum, stratum spinosum, and stratum basale.

### 1. Stratum corneum

The stratum corneum is the outermost layer of the epidermis, consisting of 4 to 8 layers of thin, flattened, dead keratinocytes stacked together.

### 2. Stratum lucidum

The stratum lucidum consists of 2 to 3 layers of flattened, anucleate, transparent keratinocytes.

### 3. Stratum granulosum

The stratum granulosum consists of 2 to 5 layers of flattened, nucleated cells with relatively thick structure. These cells are non-dividing and nearly dead, with keratohyalin granules visible within the cells. The layer functions to refract light and reduce penetration of ultraviolet radiation into the body.

### 4. Stratum spinosum

The stratum spinosum is the thickest layer of the epidermis, consisting of 4 to 10 layers of polygonal cells with spine-like structure. Intercellular spaces are present between the cells in the layer. Glycosaminoglycans are present between the cells. These sugars are hydrophilic, facilitating exchange of substances with the surrounding environment. The cytoplasm also contains granular phospholipids and acidic mucopolysaccharides, serving as sites for lymphatic circulation and exchange of substances. The layer contains numerous sensory nerve endings and tissue fluid that enables perception of various external stimuli, while also providing nutrients to the cells.

### 5. Stratum basale

The stratum basale is the innermost layer of the epidermis, connected to the dermis in an undulating pattern and consisting of 2 to 4 layers of cells with different shapes. The layer is responsible for epidermal proliferation, with newly proliferated cells migrating outward and gradually differentiating into other layers of the epidermis.

### (II) Dermis

The cellular components of the dermis include fibroblasts, macrophages, and mast cells [2]. Collagen fibers, elastic fibers, and extracellular matrix are secreted and produced by fibroblasts. Reticular fibers are immature collagen fibers rather than an independent component. The thickness of the dermis is closely related to the amount of fibrous tissue and extracellular matrix it contains, and is also closely associated with skin density, plumpness, laxity, and wrinkling, which has garnered increasing attention from cosmetic dermatologists in recent years.
1. Collagen fibers are the most abundant components in the connective tissue of the dermis. Beneath the epidermis, collagen fibers around epidermal appendages and blood vessels are fine and lack a specific orientation, while collagen fibers in other areas of the dermis are bundled together. Collagen fibers are finer in the upper dermis and thicker in the lower dermis. In the middle and lower dermis, collagen bundles are oriented almost parallel to the skin surface and interwoven with each other, extending in various directions on a horizontal plane. Collagen fibers are considered the dermal component most closely associated with skin aging.
2. Reticular fibers may be regarded as newly formed, fine collagen fibers. They are the earliest fibers to appear during embryonic development. In normal adult skin, reticular fibers are sparse and only observed beneath the epidermis, and around sweat glands, sebaceous glands, hair follicles, and capillaries. Reticular fibers beneath the epidermis are arranged in a reticular pattern. Each adipocyte is also surrounded by reticular fibers. Under conditions such as wound healing or pathological conditions with active fibroblast proliferation and new collagen formation, reticular fibers may undergo marked proliferation.
3. Elastic fibers are much thinner than collagen fibers and exhibit a wavy appearance. Elastic fibers are thickest in the dermis, arranged in the same direction as collagen bundles, and may intertwine among collagen bundles in parallel to the epidermis. In the dermal papillae beneath the epidermis, fine elastic fibers extend almost vertically upward and terminate below the epidermal-dermal junction. Elastic fibers are primarily closely associated with skin elasticity.
4. The extracellular matrix is an amorphous substance that fills the spaces between collagen fibers and collagen bundles. In normal dermis, the matrix mainly contains non-sulfated acidic mucopolysaccharides, such as hyaluronic acid (also known as hyaluronan). Although present in small amounts in normal skin, hyaluronic acid can absorb up to 1,000 times its weight in water, thereby playing an important role in anti-wrinkle and anti-aging functions of the skin.
5. Cells: The dermis contains fibroblasts, mast cells, histiocytes, lymphocytes, and a small number of dermal dendritic cells, melanophages, and Langerhans cells. Fibroblasts are capable of producing collagen fibers, elastic fibers, reticular fibers, and extracellular matrix. In addition, fibroblasts serve as the primary cells for tissue repair in the dermis following deep tissue injury.

### (III) Subcutaneous tissue

Subcutaneous tissue refers to the loose connective tissue and adipose tissue beneath the skin, connecting the skin to the underlying muscles, and is commonly referred to as superficial fascia. Located between the skin and deep tissue, the subcutaneous tissue provides the skin with a certain degree of mobility.

The subcutaneous tissue consists of loose connective tissue and fat lobules, which is connected superiorly to the dermis and inferiorly to fascia, aponeuroses, or periosteum. Adipocytes possess transparent cytoplasm with nuclei displaced toward the periphery. Adipocytes aggregate to form primary lobules, and multiple primary lobules assemble into secondary lobules, surrounded by fibrous septa, also referred to as trabeculae. The adipose septa contain blood vessels, lymphatic vessels, nerves, eccrine sweat glands, and apocrine sweat glands.

In one example, the first protease is selected from any one or more of the following:
epidermal-specific kallikreins (KLKs, human kallikrein-related peptidases) (e.g., KLK5, KLK6, KLK7), SASPase, Caspase-14, and Furin protease.

Epidermal-specific kallikreins (KLKs), belonging to the serine protease family, are expressed in the stratum granulosum. KLKs are secretory trypsin-like serine proteases that appear to be most abundantly expressed in human skin, particularly in the upper stratum spinosum and stratum granulosum, where keratinocytes undergo terminal differentiation and transform into flattened, brick-like structures to form the stratum corneum, the outermost epidermal layer and a barrier to the external environment [1].
[1] Debela et al., J Mol Biol, 373, 1017-1031 (2007); Tan et al., J Med Chem. 2015 Jan 22; 58(2): 598-612 (2014).

KLKs are encoded by 15 distinct genes, each containing 5 exons and arranged in tandem on human chromosome 19. The 15 human KLK genes are located on chromosome 19q13.4, forming the largest contiguous serine protease cluster in the human genome. These genes typically consist of five coding exons, and in some cases one or two 5' noncoding exons, encoding the kallikrein-related peptidases KLK1 to KLK15. All KLK genes encode single-chain zymogens containing chymotrypsin- or trypsin-like catalytic domains with 224 to 237 residues [2].

[2] Debela et al., Biol Chem 389, 623-632 (2008).

Among the 15 members of the KLK family, 11 are expressed in the skin, with their expression limited to the epidermis and skin appendages. Gene expression analysis indicate that KLK1 and KLK11 are the most highly expressed KLKs in the skin. KLK4, KLK5, KLK6, KLK7, and KLK13 are moderately expressed, while KLK8 mRNA is rarely detected under steady-state conditions [3]. With respect to expression sites, KLK6, KLK9, KLK10, KLK13, and KLK14 mRNAs appear strictly confined to the stratum granulosum of healthy epidermis, while KLK1, KLK4, and KLK11 mRNAs are present in both the stratum spinosum and stratum basale of keratinocytes.

Most KLK genes expressed in the epidermis are also expressed in skin appendages [3]. At the protein level, KLKs generally exhibit broader distribution in the epidermis. KLK5 is present in the stratum granulosum and stratum corneum, while KLK6 is distributed throughout the epidermis but is enriched only in the upper stratum granulosum. KLK7 is detected in both the stratum basale and upper stratum granulosum of the epidermis, but is scarcely detected in the intervening regions.

[3] N. Komatsu, M. Takata, N. Otsuki, T. Toyama, R. Ohka, K. Takehara, K. Saijoh, Expression and localization of tissue kallikrein mRNAs in human epidermis and appendages, J. Invest. Dermatol. 121 (2003) 542-549. https://doi.org/10.1046/j.1523-1747.2003.12363.x.

Additionally, KLK8 and KLK13 have been observed in the stratum corneum and stratum granulosum, as well as in appendages of healthy skin [4, 5]. Thus, the distribution of proteins is broader than that of gene expression, indicating diffusion of KLKs in the epidermis. Among all KLKs, KLK14 appears to be the most specific to the skin, being present at low levels in other tissues but at relatively high levels in the skin, with a level of 250 ng/g of total protein [6, 7].

[4] N. Komatsu, K. Saijoh, T. Toyama, R. Ohka, N. Otsuki, G. Hussack, K. Takehara, E.P. Diamandis, Multiple tissue kallikrein mRNA and protein expression in normal skin and skin diseases, Br. J. Dermatol. 153 (2005) 274-281. https://doi.org/10.1111/j.1365-2133.2005.06754.x.

[5] M. Kishibe, Y. Bando, R. Terayama, K. Namikawa, H. Takahashi, Y. Hashimoto, A. Ishida-Yamamoto, Y. Jiang, B. Mitrovic, D. Perez, H. Iizuka, S. Yoshida, Kallikrein 8 is involved in skin desquamation in cooperation with other kallikreins, J. Biol. Chem. 282 (2007) 5834-5841. https://doi.org/10.1074/jbc.M607998200.

[6] J.L.V. Shaw, E.P. Diamandis, Distribution of 15 human kallikreins in tissues and biological fluids, Clin. Chem. 53 (2007) 1423-1432. https://doi.org/10.1373/clinchem.2007.088104.

[7] C.A. Borgoño, I.P. Michael, J.L.V. Shaw, L. Luo, M.C. Ghosh, A. Soosaipillai, L. Grass, D. Katsaros, E.P. Diamandis, Expression and functional characterization of the cancer-related serine protease, human tissue kallikrein 14, J. Biol. Chem. 282 (2007) 2405-2422. https://doi.org/10.1074/jbc.M608348200.

SASPase is a protease identified in human skin with a molecular weight of 28 kDa. SASPase is specifically expressed in the stratum granulosum of human skin and undergoes autolysis to generate a 14 kDa protein. Recombinant SASPase has been reported to exhibit autolytic activity as well as the ability to degrade exogenous substrates, such as insulin and casein. However, its function in the epidermis has not been determined [8].

[8] Bernard D. et al., J. Invest. Dermatol. 125, 278-287, 2005.

The caspase family belongs to cysteine proteases and is equivalent to ced-3 in *C*. *elegans,* with high homology. These proteases share the following characteristics: (i) They invariably cleave substrates after aspartic acid residues, hence named caspases (cysteine aspartate-specific proteases). (ii) Their enzymatic activity depends on the nucleophilicity of cysteine residues. (iii) They typically exist in the form of zymogens, forming heterotetramers composed of two large and two small subunits. The large and small subunits are encoded by the same gene, and upon activation, the precursor is cleaved to generate two active subunits. These proteases are key enzymes that trigger apoptosis. Once the signaling transduction pathway is activated, caspases are activated, followed by a cascade reaction of apoptotic proteases. Activated intracellular enzymes can degrade essential cellular proteins, ultimately leading to irreversible apoptosis.

Caspase-14 (CASP14), a member of the caspase family, plays a crucial role in the formation and regulation of the stratum corneum.

Furin protease is an intracellular endoprotease that proteolytically activates a large number of precursor protein substrates in secretory pathway compartments by cleaving clusters of basic residues, typically in the form of RX(K/R)R.

In one example, the first enzyme cleavage site comprises an amino acid sequence having at least 50%, 60%, 85%, 90%, 99%, or 100% identity to any one of SEQ ID NOs: 1 to 4, SEQ ID NO: 50 (RXXR), and SEQ ID NO: 51 (RRXX).

Herein, in SEQ ID NO: 5049 and SEQ ID NO: 51, "X" in RXXR and RRXX is any amino acid. Preferably, "XX" in RXXR is RK, TK, or TR; "XX" in RRXX is SV or KR.

In one example, the single-chain polypeptide provided by the present disclosure is characterized by further comprising one or more of a signal peptide for increased expression, a quantitative protein for quantification, and a tag protein.

Further, one or more of the signal peptide, the quantitative protein, and the tag protein are respectively subjected to the following corresponding limitations:
the signal peptide comprises an amino acid sequence having at least 85%, 90%, 99%, or 100% identity to SEQ ID NO: 24;
the quantitative protein is a fluorescent protein;
the tag protein is one or more of a His tag, a GST tag, an MBP tag, and a Strep-tagII tag, or comprises an amino acid sequence having at least 85%, 90%, 99%, or 100% identity to SEQ ID NO: 25.

The phrase "one or more of the signal peptide, the quantitative protein, and the tag protein are respectively subjected to the following corresponding limitations" is understood as follows. For example, only the signal peptide among the signal peptide, the quantitative protein, and the tag protein is subjected to the corresponding limitation described above. For another example, the signal peptide, the quantitative protein, and the tag protein are each respectively subjected to their corresponding limitations described above. For yet another example, any two of the signal peptide, the quantitative protein, and the tag protein are respectively subjected to their corresponding limitations described above, and so forth.

In one example, the single-chain polypeptide further comprises two second enzyme cleavage sites located at the N-terminus of the first domain and the C-terminus of the second domain, respectively, and a second protease that cleaves the second enzyme cleavage site is incapable of cleaving the first enzyme cleavage site. Thus, by cleavage of the two second enzyme cleavage sites, portions of the single-chain polypeptide other than the first domain, the first enzyme cleavage site, and the second domain can be removed, e.g., removing the signal peptide, quantitative protein, and tag protein, to obtain a single-chain polypeptide comprising only the first domain, the first enzyme cleavage site, and the second domain.

In one example, the first enzyme cleavage site and/or the second enzyme cleavage site are typically not cleaved by proteases encountered during expression of the single-chain polypeptide within a host cell or during synthesis of the single-chain polypeptide in an *in vitro* synthesis system involving a cell extract derived from the host cell. That is, when the single-chain polypeptide is obtained *in vivo* within a host cell, or when the single-chain polypeptide is synthesized *in vitro* using a cell extract derived from the host cell, the first enzyme cleavage site and/or the second enzyme cleavage site are typically not cleaved by proteases encountered in these two methods. Thus, when the first enzyme cleavage site satisfies the premise, it is not readily activated during production, ensuring a certain level of production safety; when the second enzyme cleavage site satisfies the premise, a certain structure of the single-chain polypeptide can be maintained as needed, e.g., retaining a tag protein to facilitate purification of the product during production of the single-chain polypeptide.

In one example, the second enzyme cleavage site is designed to be highly specific for the second protease. That is, as described above, the second enzyme cleavage site has a one-to-one correspondence with the second protease, such that under most normal circumstances (*i.e.,* typically), the second enzyme cleavage site can only be cleaved by the specific second protease and is not randomly cleaved during production, thereby maintaining a certain structure of the single-chain polypeptide before cleavage at the second enzyme cleavage site.

Further, the second protease is not an enzyme that is commonly encountered, e.g., not a protease in the human body. As used herein, the protease in the human body refers to a protease that is not typically present in the human body. Thus, the likelihood of cleavage due to contact with proteases in the human body during production is reduced, thereby preventing undesired structural changes at inappropriate stages.

In one example, preferably, the second protease is selected from a non-human enterokinase, a tobacco etch virus protease, a protease derived from *Bacillus subtilis, a* protease derived from *Bacillus amyloliquefaciens,* a protease derived from rhinovirus, papain, a homolog of papain of an insect, or a homolog of papain of a crustacean.

In one example, the second enzyme cleavage site comprises an amino acid sequence having at least 85%, 90%, 99%, or 100% identity to any one of SEQ ID NOs: 5 to 9. In SEQ ID NO: 6, "X" in EXXYXQS/G refers to any amino acid.

In one example, when the second domain comprises an amino acid sequence having at least 80%, 85%, 90%, 95%, 99%, or 100% identity to SEQ ID NO: 17, the lysine at the position corresponding to position 423 of SEQ ID NO: 17 in the amino acid sequence of the second domain is mutated to a non-lysine residue. For example, the lysine is mutated to asparagine, glutamic acid, or alanine, thereby preventing the second domain from being inactivated due to cleavage by Trypsin.

As used herein, for an amino acid sequence that is completely identical (*i.e.,* 100% identity) to SEQ ID NO: 17, the lysine at the position corresponding to position 423 refers to the lysine at position 423; for other amino acid sequences that are not completely identical to SEQ ID NO: 17, the lysine at the position corresponding to position 423 refers to the lysine located at a position in the amino acid sequence that is within a reasonable misalignment range relative to position 423 of SEQ ID NO: 17. The reasonable misalignment range means that, for those skilled in the art, when the amino acid sequence has at least 80% identity (*i.e.,* homology) to the reference amino acid sequence, the residue numbering may be misaligned toward the N-terminus or C-terminus relative to the reference amino acid sequence, and such a misalignment is acceptable.

For example, compared to SEQ ID NO: 17, when an amino acid sequence has at least 80% identity to SEQ ID NO: 17, and the amino acid is lysine located at a position misaligned by 5 residues relative to position 423 of SEQ ID NO: 17, such a shift of 5 residues falls within the acceptable shift range. Accordingly, those skilled in the art can determine that the lysine at position 427 corresponds to the lysine at position 423.

In one example, the intermediate amino acid sequence region further comprises XTXS, wherein the X is an amino acid other than K; preferably, the X is Q.

In a preferred example, the structure of the intermediate amino acid sequence region from the N-terminus to the C-terminus is:
XTXS-linker (L3)-at least one first enzyme cleavage site; similarly, when there are multiple first enzyme cleavage sites, all of the first enzyme cleavage sites are identical or different from one another; preferably, the multiple first enzyme cleavage sites are sequentially connected from the N-terminus to the C-terminus. Further, the number of amino acids in L3 is 0 to 20, 0 to 10, or 0 to 5.

As used herein, the connection among the first domain, the intermediate amino acid sequence region (when the intermediate amino acid sequence region comprises only the first enzyme cleavage site), and the second domain is not particularly limited, and is preferably in the order from the N-terminus to the C-terminus.

### 2. Application

The single-chain polypeptide described above provided by the present disclosure is used in any one or more of the following: skin care, plastic surgery, body contouring, and therapy by inducing muscle relaxation or relief of muscle spasm.

Specifically, the present disclosure further provides a product or medicament comprising the single-chain polypeptide described above; preferably, the product comprising the single-chain polypeptide is used in any one or more of the following: skin care, plastic surgery, body contouring, and therapy by inducing muscle relaxation or relief of muscle spasm.

In addition, the product or medicament comprising the single-chain polypeptide described above may be administered at a specific site by means of, *e.g.,* topical application, spraying, external application, or injection. For example, when administered by topical application into skin tissue, the first cleavage site is cleaved by a specific first protease at the specific site, thereby converting the single-chain polypeptide into an activated form to exert the effect.

3. The present disclosure further provides a nucleic acid, comprising a nucleotide sequence encoding the single-chain polypeptide described above.

The present disclosure further provides a vector comprising the nucleic acid described above.

The present disclosure further provides a host cell comprising the nucleic acid described above or the vector described above. Specifically, the host cell is derived from a prokaryotic cell or a eukaryotic cell; further, the cell is selected from the following groups: one or more of an *Escherichia coli* cell, a human-derived cell, a Chinese hamster ovary cell, an insect cell, a wheat germ cell, a rabbit reticulocyte, and a yeast cell, or a combination thereof.

Further, the host cell is derived from a yeast cell; further, the yeast cell is selected from one or more of *Saccharomyces cerevisiae* and *Kluyveromyces,* or a combination thereof; in another preferred example, the *Kluyveromyces* is selected from one or more of *Kluyveromyces lactis, Kluyveromyces marxianus,* and *Kluyveromyces dobzhanskii,* or a combination thereof.

The present disclosure further provides an *in vitro* cell-free synthesis system, comprising synthesizing the single-chain polypeptide using an mRNA or DNA encoding the single-chain polypeptide described above as a template.

In one example, the *in vitro* cell-free synthesis system comprises any one or more of the following:
**(1)** an mRNA or DNA template encoding the single-chain polypeptide described above;
(2) a cell extract, wherein the cell extract is a yeast cell extract:
   in one example, the cell extract is derived from a strain selected from the group consisting of *Saccharomyces cerevisiae, Pichia pastoris,* and *Kluyveromyces,* or a combination thereof; preferably, the yeast cell extract is derived from *Kluyveromyces,* more preferably *Kluyveromyces lactis*;
(3) any one or more of the following components:
   an amino acid mixture, dNTP, RNA polymerase, DNA polymerase, an energy supply system, polyethylene glycol, and an aqueous solvent.

### 4. Preparation method for single-chain polypeptides:

The single-chain polypeptide is obtained by performing an *in vitro* synthesis reaction using an mRNA or DNA encoding the single-chain polypeptide described above as a template.

In one example, the *in vitro* synthesis reaction is performed in a reaction system comprising the *in vitro* cell-free synthesis system described above; preferably, the DNA template and other components involved in the *in vitro* synthesis reaction have a volume ratio of 1:10 to 1:50, or 1:20 to 1:40, or 1:25 to 1:35, or 1:30.

In one example, the *in vitro* cell-free synthesis system comprises a yeast cell extract, an amino acid mixture, dNTP, RNA polymerase, DNA polymerase, an energy supply system, polyethylene glycol, and an aqueous solvent.

In one example, the yeast cell extract is derived from a strain selected from the group consisting of *Saccharomyces cerevisiae, Pichia pastoris,* and *Kluyveromyces,* or a combination thereof; preferably, the yeast cell extract is derived from *Kluyveromyces,* more preferably *Kluyveromyces lactis.*

In one example, the yeast cell extract accounts for 50 to 80% of the total volume of the *in vitro* cell-free synthesis system.

The present disclosure enables the expression and purification of the single-chain polypeptide described above through the *in vitro* synthesis reaction described above, which is characterized by a short cycle and convenient operation. Moreover, since the method employs an *in vitro* preparation strategy with the addition of mRNA or DNA templates, only a DNA template of a plasmid carrying the modified gene is required, eliminating the risk of toxic strain leakage.

In one example, the preparation method for the single-chain polypeptide further comprises: digesting the obtained single-chain polypeptide or the purified single-chain polypeptide with the second protease to obtain a single-chain polypeptide comprising only the first domain, the first enzyme cleavage site, and the second domain.

In the present disclosure, the *in vitro* cell-free synthesis system comprises an *in vitro* translation system, an *in vitro* transcription-translation system (IVTT system), *etc.*

The term *"in vitro* protein synthesis reaction" refers to the synthesis of proteins in an *in vitro* cell-free synthesis system, which at least includes the translation process. The term includes, but is not limited to, an IVTT reaction (*in vitro* transcription-translation reaction). In the present disclosure, the IVTT reaction is preferred. The IVTT reaction, corresponding to an IVTT system, is a process of transcribing and translating DNA into protein *in vitro.* Accordingly, such *in vitro* protein synthesis systems are also referred to as the D2P system, D-to-P system, D_to_P system, or DNA-to-Protein system. The corresponding *in vitro* protein preparation methods are also referred to as the D2P method, D-to-P method, D_to_P method, or DNA-to-Protein method.

In the *in vitro* synthesis reaction of the examples of the present disclosure, the *in vitro* cell-free synthesis systems, templates, plasmids, target proteins, *in vitro* synthesis reactions (incubation reactions), various preparation methods, various detection methods as technical elements of the present disclosure may also be independently selected from the suitable embodiments or implementation methods disclosed in the following references, including, but not limited to, CN111484998A, CN106978349A, CN108535489A, CN108690139A, CN108949801A, CN108642076A, CN109022478A, CN109423496A, CN109423497A, CN109423509A, CN109837293A, CN109971783A, CN109988801A, CN109971775A, CN110093284A, CN110408635A, CN110408636A, CN110551745A, CN110551700A, CN110551785A, CN110819647A, CN110845622A, CN110938649A, and CN110964736A. Unless conflicting with the purpose of the present disclosure, these references and their cited references are incorporated by reference in their entirety and for all purposes.

The present disclosure is further described below based on botulinum toxin type A and in combination with various experimental examples.

### Example 1: Preparation of DNA template

### 1. Construction of target plasmids

(1) Construction of a target plasmid containing a nucleotide sequence encoding a protein structure of formula I:
   SP-8His-F2-LC-D(KTKS-F1)-HC-F2-EGFP-8His (formula I, connected from the N-terminus to the C-terminus);
   in the formula, D represents the intermediate amino acid sequence region, KTKS represents a portion of the natural botulinum toxin type A cleavage site, F1 represents the first enzyme cleavage site designed in the present disclosure, corresponding to different first proteases, including KLK5, KLK7, SASPase, and Caspase-14;
   F2 represents the second enzyme cleavage site, corresponding to the second protease, which in this example is TEV protease;
   SP represents a signal peptide, 8His represents a tag protein; EGFP represents a fluorescent protein for quantification;
   LC represents the first domain, whose amino acid sequence is shown in SEQ ID NO: 10 in this example; HC represents the second domain, whose amino acid sequence is shown in SEQ ID NO: 17 in this example;
   The lysine at position 423 of HC is mutated to asparagine.

The primers listed in Table 1 correspond to nucleotide sequences encoding the protein structures described above corresponding to different first proteases.

According to each primer pair listed in Table 1, correct sequences were obtained by PCR using the QuickChange method, followed by transformation to generate different target plasmids.

| Table 1 | |
|---|---|
| Primer name | 5'-3' sequence |
| KLK5-F | TACAGATCTAGAATCGTT GCTTTGAACGACTTGTGTATC |
| KLK5-R | AACGATTCTAGATCTGTAAGACTTGGTCTTAGAGGTGATG |
| KLK7-F | AAGCACTRGTACTCTAAC GCTTTGAACGACTTGTGTATC |
| KLK7-R | GTTAGAGTACAAGTGCTTAGACTTGGTCTTAGAGGTGATG |
| SASPase-F | GTTTCTCAAGTTTACCCAATCGTTCAA GCTTTGAACGACTTGTGTATC |
| SASPase-R | TGGGTAAACTTGAGAAACAGACTTGGTCTTAGAGGTGATG |
| Caspase-14-F | GGTGGTTGGGAACACGACGGTATCGCTTTC GCTTTGAACGACTTGTGTATC |
| Caspase-14-R | GTGTTCCCAA CCACCAGACT TGGTCTTAGA GGTGATG |

(2) For the protein structure of formula I described above, in the natural botulinum toxin type A, lysine residues at positions 438 and 440 of LC were mutated to alanine, thereby constructing corresponding modified target plasmids. That is, the intermediate amino acid sequence region D was modified to QTQS-F1.

The primers listed in Table 2 correspond to nucleotide sequences encoding the modified protein structures corresponding to different first proteases.

Correct sequences were obtained by PCR using the QuickChange method, followed by transformation to generate different target plasmids.

| Table 2 | |
|---|---|
| Primer name | 5'-3' sequence |
| KLK5-2Q-F | CTCAGACCCAGTCTGGTGGT TACAGATCTAGAATCGTT GCTTTGAACGAC |
| KLK5-2Q-R | ACCAGACTGGGTCTGAGAGGTGATGATACCTCTAACACAC |
| KLK7-2Q-F | CTCAGACCCAGTCTGGTGGT AAGCACTTGTACTCTAAC GCTTTGAACGAC |
| KLK7-2Q-R | ACCAGACTGGGTCTGAGAGGTGATGATACCTCTAACACAC |
| SASPase-2Q-F | CTCAGACCCAGTCT GGTGGT GTTTCTCAAGTTTACCCAATCGTTCAA GC |
| SASPase-2Q-R | ACCAGACTGGGTCTGAGAGGTGATGATACCTCTAACACAC |
| Caspase-14-2Q-F | CTCAGACCCAGTCTGGTGGT TGGGAACAC |
| Caspase-14-2Q-R | ACCAGACTGGGTCTGAGAGGTGATGATACCTCTAACACAC |

### (3) Plasmid transformation

### Transformation of the target plasmids was performed as follows:

1 µL of the target plasmid was added to 20 µL of DH5α, placed on ice for 30 minutes, and heat-shocked at 42°C for 45 seconds. The mixture was placed on ice for 2 minutes, added with 500 µL of medium, and incubated at 37°C with shaking at 200 rpm for 1 hour. 100 µL of the culture was pipetted onto an LB plate (containing antibiotic) and incubated at 37°C in an inverted position for 12 to 16 hours. The plate was then removed and stored in a 4°C refrigerator.

### (4) Extraction and amplification of transformed plasmids

Amplification system: random primers at a final concentration of 20 to 30 µM, plasmid template at 0.05 to 0.15 µg/mL, 0.5 to 1 mM dNTP, 2× BSA, 1× phi29 reaction buffer (comprising 50 mM Tris-HCl, 10 mM MgCl₂, 10 mM (NH₄)₂SO₄, 4 mM DTT, pH 7.5);

### Taking a 10 mL amplification system as an example:

10 mL of the amplification system, plasmid at a final concentration of 4 ng/µL, and 5 µL of phi29 DNA polymerase at 0.05 to 0.1 mg/mL were mixed and reacted at 30 rpm for 2 hours at 37°C. The product was then verified by 1% agarose gel electrophoresis.

### Example 2: In vitro cell-free protein synthesis (IVTT)

The *in vitro* cell-free protein synthesis system comprised: 22 mM HEPES (pH 7.4), 30 to 150 mM potassium acetate, 1.0 to 5.0 mM magnesium acetate, 1.5 to 4 mM nucleoside triphosphate mixture (ATP, GTP, CTP, and UTP), 0.08 to 0.24 mM amino acid mixture (glycine, alanine, valine, leucine, isoleucine, phenylalanine, proline, tryptophan, serine, tyrosine, cysteine, methionine, asparagine, glutamine, threonine, aspartic acid, glutamic acid, lysine, arginine, and histidine), 25 mM phosphocreatine, 1.7 mM dithiothreitol, 0.27 mg/mL creatine phosphokinase, 0.027 to 0.054 mg/mL T7 RNA polymerase, 1% to 4% polyethylene glycol, 0.5% to 2% sucrose, and finally 50% to 80% (v/v) yeast cell extract.

The yeast cell extract in this example was derived from *Kluyveromyces lactis.*

More specifically, the IVTT reaction was performed using the Standard product from the Protein Factory of Kangma-Healthcode (Shanghai) Biotech Co., Ltd.

The amplification product (DNA template) obtained in Example 1 was added to the *in vitro* cell-free protein synthesis system at a volume ratio of 1:30. The mixture was incubated at 100 rpm in an incubator at 30 ± 2°C for 3 to 6 hours.

### Example 3: Purification of in vitro synthesized protein according to the following steps

(1) The volume of magnetic beads was determined to be 1% of the total volume of the *in vitro* cell-free protein synthesis system.
(2) The beads were washed three times with water before use, vortexed for 15 seconds each time, and collected using a magnetic rack. The volume of water used each time was 5 to 10 times the volume of the beads.
(3) Binding of target protein to beads: The washed beads were mixed with the IVTT product in a large beaker and incubated at 100 rpm for 1 hour at 4°C. The beads were then collected into a centrifuge tube.
(4) Washing of impurities: The protein-bound beads were washed three times with 30 mM imidazole at 180 rpm for 10 minutes each time. The volume of wash buffer used each time was 3 to 5 times the volume of the beads.
(5) Elution was performed with 280 mM imidazole elution buffer. The volume of the elution buffer was twice the volume of the beads.
(6) Centrifugation was performed at 12,000 rpm for 5 minutes each time to remove residual beads.

The elution in steps (4) to (5) was performed at pH 8.0.

### Example 4: Electrophoresis analysis of purification results

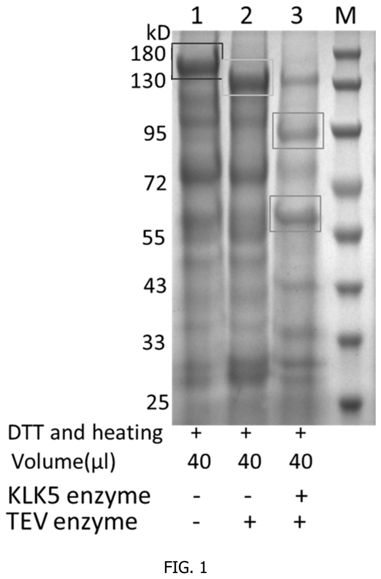

The electrophoresis results of the modified protein with KLK5 as the first protease are shown in FIG. 1.

In FIG. 1, "+" indicates that the corresponding treatment was applied, and "-" indicates absence of the corresponding treatment. Disulfide bond reducing agents are able to reduce disulfide bonds. After reduction of the disulfide bond between the first domain and the second domain of the activated single-chain polypeptide, two fragments corresponding to the first and second domains were generated, with molecular weights of 50 kDa and 100 kDa, respectively. Successful reduction of the disulfide bond indicated that the *in vitro* synthesized protein was obtained as a single-chain polypeptide capable of activation.

As can be seen from FIG. 1, after modification, *i.e.,* lysine residues at positions 438 and 440 of LC were mutated to alanine (the intermediate amino acid sequence region was modified to (QTQS-F1)), the results were as follows:

Purification at pH 8.0 yielded the corresponding protein. Before cleavage, even in the presence of a disulfide bond reducing agent, a band greater than 150 kDa was observed, indicating that the corresponding protein was obtained and that no activation occurred when the first enzyme cleavage site was not cleaved.

After cleavage of only the second enzyme cleavage site, followed by reduction of the disulfide bond, a band at approximately 150 kDa was observed, indicating that cleavage at the second enzyme cleavage site was successful, resulting in a polypeptide comprising only the first domain, the first enzyme cleavage site, and the second domain, and that no activation occurred when the first enzyme cleavage site was not cleaved.

After cleavage of both the first and second enzyme cleavage sites, followed by reduction in the presence of a disulfide bond reducing agent, two bands at 100 kDa and 50 kDa was observed, indicating that compared with the above, cleavage of the first enzyme cleavage site successfully converted the polypeptide into the activated form of a double-chain structure, in which the first and second domains are connected by a disulfide bond.

Prior to modification, the elution showed no purified protein bands, indicating that the retained natural cleavage sites were prone to cleavage.

### Example 5: Enzymatic digestion method

For purified proteins obtained from the preceding examples:
TEV single digestion: The purified protein was digested overnight with TEV protease at 20 to 30°C with the addition of 3 mM GSH and 0.3 mM GSSG.
Double digestion: The purified protein was digested with KLK5 protease at 20 to 30°C for at least 8 hours, followed by the addition of 3 mM GSH and 0.3 mM GSSG, and digested overnight with TEV protease at 20 to 30°C.

### Example 6: In vitro activity assay of enzymatic digestion products

To detect the activity of digestion products *in vitro* in a simple, efficient, and accurate manner, the fluorescence resonance energy transfer (FRET) method was adopted, wherein photon energy can be transferred from an excited fluorophore (donor) to another fluorophore (acceptor) when the donor and acceptor are in close proximity (1 to 10 nm). In this experiment, a substrate system with a CFP-substrate cleavage site-YFP structure was employed. Under normal conditions, excitation at 433 nm results in an emission signal at 527 nm (yellow fluorescence). When active digestion products are present, the substrate system is cleaved by the digestion products, and excitation at 433 nm results in an emission signal at 476 nm (blue fluorescence). The cleavage activity of the digestion products on the substrate can thus be calculated based on the change in the ratio of blue fluorescence to yellow fluorescence.
(I) Activity effects of double digestion products on different substrates
   **1. Experimental objective:** To evaluate the cleavage effect of the digestion products on the substrate proteins CFP-SNAP25(17aa)-YFP and CFP-SNAP25(66aa)-YFP.
   **2. Experimental procedure**
      2.1 Reagents
         2.1.1 HEPES;
         2.1.2 NaCl;
         2.1.3 DTT;
         2.1.4 Zn(OAc)₂;
         2.1.5 Purified water;
         2.1.6 CFP-SNAP25(17aa)-YFP, 1.9 mg/mL (55.72 kDa);
         2.1.7 CFP-SNAP25(66aa)-YFP, 2.8 mg/mL (61.22 kDa);
         2.1.8 Double digestion product, 700 µg/mL;
         2.1.9 Na₂CO₃.
      2.2 Consumables
         2.2.1 50 mL centrifuge tube;
         2.2.2 384-well microplate;
         2.2.3 200 µL pipette tip;
         2.2.4 10 µL pipette tip;
         2.2.5 1.5 mL EP tube.
      2.3 Equipment
         2.3.1 Microplate reader;
         2.3.2 1 mL pipette;
         2.3.3 200 µL pipette;
         2.3.4 10 µL pipette;
         2.3.5 pH meter;
         2.3.6 Analytical balance.
      2.4 Assay procedure for substrate cleavage
         2.4.1 Preparation of cleavage reaction buffer: 0.1835 g of Zn(OAc)₂ was first weighed using an analytical balance, followed by the addition of ultrapure water to make up the volume to 50 mL, resulting in a 20 mM Zn(OAc)₂ solution for later use.
         2.4.2 0.5958 g of HEPES, 0.0146 g of NaCl, and 0.0154 g of DTT were weighed using an analytical balance and placed into a 50 mL centrifuge tube, added with 50 µL of the 20 mM Zn(OAc)₂ solution, followed by the addition of ultrapure water to make up the volume to approximately 45 mL, and mixed well.
         2.4.3 Subsequently, the mixture was added with a 1 M Na₂CO₃ solution to adjust the pH to 7.4. Finally, the cleavage buffer was brought to volume in a 50 mL volumetric flask, resulting in a cleavage reaction system for later use.
         2.4.4 Both substrate proteins were thawed on ice.
         2.4.5 The microplate reader and its control computer were turned on, and the temperature in the reaction chamber was set to 37°C.
         2.4.6 Each cleavage reaction was performed in a total volume of 50 µL, with different substrate-to-double digestion product ratios (concentration ratios) set as follows: 8:1; 5:1; 2.5:1.
         2.4.7 The final reaction system was prepared in a 1.5 mL EP tube according to the ratios described above: In the reaction system with a substrate-to-double digestion product ratio of 8:1, the double digestion product was at a concentration of 140 µg/mL and the substrate was at a concentration of 1120 µg/mL. In the reaction system with a substrate-to-double digestion product ratio of 5:1, the double digestion product was at a concentration of 140 µg/mL and the substrate was at a concentration of 700 µg/mL. In the reaction system with a substrate-to-double digestion product ratio of 2.5:1, the double digestion product was at a concentration of 140 µg/mL and the substrate was at a concentration of 350 µg/mL. The total volume was 50 µL. After addition of the substrate and double digestion product, the mixture was supplemented with cleavage reaction buffer to the final volume and mixed well. Two blank controls containing only CFP-SNAP25(17aa)-YFP or CFP-SNAP25(66aa)-YFP but no double digestion product were set up.
         2.4.8 After mixing, the final reaction system was added to a 384-well microplate, which was shaken in the microplate reader for 2 minutes.
         2.4.9 After shaking, the reaction program was adjusted to record readings every 2 minutes for 3 hours. The reading settings were as follows: excitation at 433 nm, emission at 476 nm; excitation at 433 nm, emission at 526 nm. Both excitation wavelengths were measured.
         2.4.10 After 3 hours of reading, the microplate was removed, and 3 µL of 0.25% Trypsin was added to each sample well to terminate the reaction. After addition of 0.25% Trypsin, the microplate was placed back into the microplate reader to start reading, with readings recorded per minute for half an hour. The reading settings were as follows: excitation at 433 nm, emission at 476 nm; excitation at 433 nm, emission at 526 nm. Both excitation wavelengths were measured.
      2.5 Cleanup
         2.5.1 Data were completely recorded.
         2.5.2 Waste disposal: All waste was disposed into a chemical waste container.
   **3. Results and analysis**

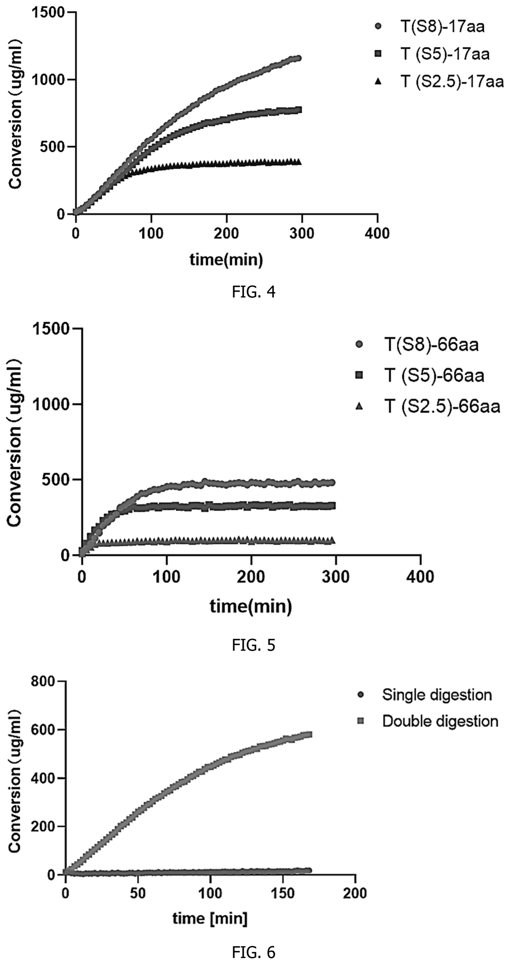

FIG. 4 and FIG. 5 show the substrate conversion rates for different concentrations of substrates CFP-SNAP25(17aa)-YFP and CFP-SNAP25(66aa)-YFP, respectively. It can be observed that there was a linear relationship between substrate conversion rate and time within a certain time range, indicating that the cleavage efficiency of the double digestion product for both substrates was as expected, preliminarily demonstrating the effectiveness of the double digestion product *in vitro.*

However, the linear relationship for CFP-SNAP25(17aa)-YFP was significantly better than that for CFP-SNAP25(66aa)-YFP, meaning that the substrate protein CFP-SNAP25(17aa)-YFP exhibited a higher conversion rate compared to CFP-SNAP25(66aa)-YFP.

In addition, as shown in FIG. 4 and FIG. 5, at substrate-to-double digestion product ratios of 8:1 and 5:1, there was a good linear relationship. Moreover, at a substrate-to-double digestion product ratio of 5:1, the reaction reached a plateau around 2 hours, whereas at a ratio of 8:1, the time required was longer. Therefore, in subsequent experiments, a substrate-to-digestion product ratio of approximately 5:1 was selected.

### (II) Effect of TEV single digestion

In this experiment, the activity effects between TEV single digestion products and double digestion products were primarily compared to exclude any effect from TEV protease.

The experimental method was the same as that in "(I) Activity effects of double digestion products on different substrates", except that only the substrate CFP-SNAP25(17aa)-YFP was used. In the system, the substrate protein CFP-SNAP25(17aa)-YFP was at a concentration of 800 µg/mL. A comparative experiment was conducted using TEV single digestion products (stock concentration of 700 µg/mL) and double digestion products, with both tested at a concentration of 140 µg/mL (substrate-to-digestion product ratio of approximately 5:1). Two blank controls containing only CFP-SNAP25(17aa)-YFP but no digestion product were set up.

As shown in FIG. 6, the double digestion product exhibited a linear relationship between substrate conversion rate and time within a certain time range, while the TEV single digestion product showed essentially no activity effect on the substrate. These experimental results indicated that residual TEV protease in the single digestion product showed no activity on the substrate, and that TEV digestion did not affect the activity of the single-chain polypeptide.

### (III) Effect of KLK5 protease

In this experiment, the activity effects between double digestion products and KLK5 protease were primarily compared to exclude any effect from KLK5 protease.
**1. Experimental objective:** To evaluate the effect of KLK5 protease
**2. Experimental procedure**
   2.1 Reagents
      2.1.1 HEPES;
      2.1.2 NaCl;
      2.1.3 DTT;
      2.1.4 Zn(OAc)₂;
      2.1.5 Purified water;
      2.1.6 CFP-SNAP25(17aa)-YFP, 26.8 mg/mL (55.72 kDa);
      2.1.7 Double digestion product, 671 µg/mL;
      2.1.8 KLK5 protease, 530 µg/mL;
      2.1.9 Na₂CO₃
      2.1.10 Tween 80.
   2.2 Consumables
      2.2.1 50 mL centrifuge tube;
      2.2.2 384-well microplate;
      2.2.3 200 µL pipette tip;
      2.2.4 10 µL pipette tip;
      2.2.5 1.5 mL EP tube.
   2.3 Equipment
      2.3.1 Microplate reader;
      2.3.2 1 mL pipette;
      2.3.3 200 µL pipette;
      2.3.4 10 µL pipette;
      2.3.5 pH meter;
      2.3.6 Analytical balance.
   2.4 Assay procedure for substrate cleavage
      2.4.1 Preparation of cleavage reaction buffer: 0.1835 g of Zn(OAc)₂ was first weighed using an analytical balance, followed by the addition of ultrapure water to make up the volume to 50 mL, resulting in a 20 mM Zn(OAc)₂ solution for later use.
      2.4.2 0.5958 g of HEPES and 0.0146 g of NaCl were weighed using an analytical balance and placed into a 50 mL centrifuge tube, added with 500 µL of the 20 mM Zn(OAc)₂ solution (resulting in a final concentration of 200 µM in the Zn(OAc)₂ solution), then added with 0.1% Tween 80 (50 µL of 100% Tween 80 in a 50 mL system), followed by the addition of ultrapure water to make up the volume to approximately 45 mL, and mixed well.
      2.4.3 Subsequently, the mixture was added with a 1 M Na₂CO₃ solution to adjust the pH to 7.4. Finally, the cleavage buffer was brought to volume in a 50 mL volumetric flask, resulting in a reaction system for later use. After bringing to volume, 10 mL of the reaction system without DTT was added to an empty tube. The appropriate amount of DTT powder was then weighed and added to the reaction system to prepare a buffer system containing 0.5 mM DTT for later use.
      2.4.4 The substrate protein CFP-SNAP25(17aa)-YFP was thawed on ice.
      2.4.5 The microplate reader and its control computer were turned on, and the temperature in the reaction chamber was set to 37°C.
      2.4.6 The final reaction system was prepared in a 1.5 mL EP tube: In the system, the substrate protein CFP-SNAP25(17aa)-YFP was at a concentration of 800 µg/mL, the double digestion product was at a concentration of 140 µg/mL, and the remaining volume was supplemented with the prepared buffer to a total of 50 µL. In the KLK5 digestion group, the substrate protein was at a concentration of 800 µg/mL, no double digestion product was added, and only KLK5 protease was added at a concentration of 1.4 µg/mL (consistent with the concentration of residual KLK5 protease in the double digestion product). Two blank controls containing only CFP-SNAP25(17aa)-YFP but no double digestion product were set up.
      2.4.7 After mixing, the final reaction system was added to a 384-well microplate, which was shaken in the microplate reader for 2 minutes.
      2.4.8 After shaking, the reaction program was adjusted to record readings every 2 minutes for 3 hours. The reading settings were as follows: excitation at 433 nm, emission at 476 nm; excitation at 433 nm, emission at 526 nm. Both excitation wavelengths were measured.
      2.4.9 After 3 hours of reading, the microplate was removed, and 3 µL of 0.25% Trypsin was added to each sample well to terminate the reaction. After addition of 0.25% Trypsin, the microplate was placed back into the microplate reader to start reading, with readings recorded per minute for half an hour. The reading settings were as follows: excitation at 433 nm, emission at 476 nm; excitation at 433 nm, emission at 526 nm. Both excitation wavelengths were measured.
   2.5 Cleanup
      2.5.1 Data were completely recorded.
      2.5.2 Waste disposal: All waste was disposed into a chemical waste container.

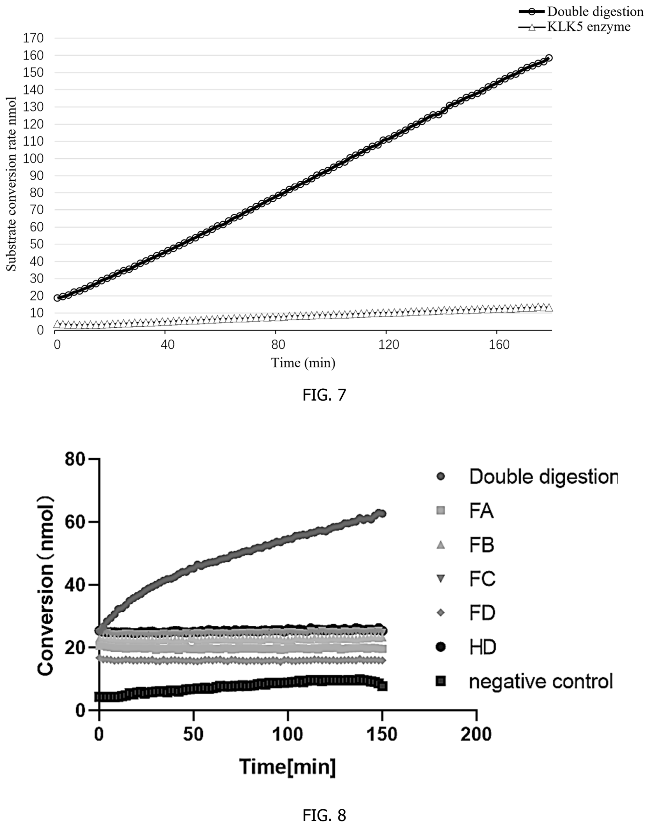

As shown in FIG. 7, the double digestion product exhibited a linear relationship between substrate conversion rate and time within a certain time range, while KLK5 protease showed essentially no activity effect on the substrate. These experimental results indicated that residual KLK5 protease in the single digestion product showed no activity on the substrate, demonstrating that the single-chain polypeptide provided by the present disclosure, after enzymatic digestion and subsequent reduction of the disulfide bond, can exert an activity effect.

### (IV) In vitro activity assay of multiple first enzyme cleavage sites

1. A structure was constructed as follows: SP-8His-F2-LC-D(F11-F12)-HC-F2-EGFP-8His.

The structure differs from formula I in that D contains two first enzyme cleavage sites as follows:

| Protein ID | Intermediate amino acid sequence region (D) |
|---|---|
| FA | RRSVGSGYRSRIV |
| FB | GSGRRKRGSGYRSRIV |
| FC | GSRRSVGGYRSRIV |
| FD | RRSVGGYRSRIV |

Botulinum proteins obtained from the structure in this example were subjected only to TEV protease digestion (single digestion), or to double digestion with TEV and KLK5 (HD). A negative control containing only KLK5 protease but no botulinum protein was set up. All groups were assayed for *in vitro* activity.

### 2. Experimental procedure

2.1 Reagents
   2.1.1 HEPES
   2.1.2 NaCl
   2.1.3 DTT
   2.1.4 Zn(OAc)₂
   2.1.5 Purified water
   2.1.6 FP1-SNAP25(17aa)-FP2, 26.8 mg/mL (55.72 kDa)
   2.1.7 Double digestion product (obtained by cleavage of both the first and second enzyme cleavage sites of HD): 671 µg/mL
   2.1.8 Single digestion product (TEV only): FA: 336 µg/mL; FB: 277 µg/mL; FC: 110 µg/mL; FD: 427 µg/mL; HD (with only the first enzyme cleavage site cleaved by KLK5): 100 µg/mL
   2.1.9 KLK5 protease, 530 µg/mL; 2.1.10 Na₂CO₃
   2.1.11 Tween 80
   2.1.12 BSA
   2.1.13 HCl
   2.1.14 PMSF
2.2 Consumables
   2.2.1 50 mL centrifuge tube
   2.2.2 384-well microplate
   2.2.3 200 µL pipette tip
   2.2.4 10 µL pipette tip
   2.2.5 1.5 mL EP tube
2.3 Equipment
   2.3.1 Microplate reader
   2.3.2 1 mL pipette
   2.3.3 200 µL pipette
   2.3.4 10 µL pipette
   2.3.5 pH meter
   2.3.6 Analytical balance
2.4 Assay procedure for Jianmeipeptide-150 enzymatic activity
   2.4.1 Preparation of Jianmeipeptide-150 digestion buffer: 0.1835 g of Zn(OAc)₂ was first weighed using an analytical balance, followed by the addition of ultrapure water to make up the volume to 50 mL, resulting in a 20 mM Zn(OAc)₂ solution for later use.
   2.4.2 0.5958 g of HEPES, 0.0146 g of NaCl, and 0.0154 g of DTT were weighed using an analytical balance and placed into a 50 mL centrifuge tube, added with 50 µL of the 20 mM Zn(OAc)₂ solution, then added with 0.1% Tween 80 (50 µL of 100% Tween 80 in a 50 mL system), followed by the addition of ultrapure water to make up the volume to approximately 45 mL, and mixed well. Additionally, other buffer systems were prepared: assay buffer system 2, in which all other conditions remained unchanged, but the Zn²⁺ concentration was increased 10-fold to 200 µM; assay buffer system 3, in which Tween 80 was omitted, and all other conditions remained unchanged, but the pH was adjusted to 5 with 1 M HCl; assay buffer system 4, in which Tween 80 was omitted, and all other conditions remained unchanged, but 1% BSA was added; assay buffer system 5, in which Tween 80 was omitted, and all other conditions remained unchanged, but 1% BSA and 1 mM PMSF were added.
   2.4.3 Subsequently, the mixture was added with a 1 M Na₂CO₃ solution to adjust the pH to 7.4 (the pH in buffer system 2 had already been adjusted to 5, so no further adjustment was needed; the pH in other systems were normally adjusted). Finally, the digestion buffer was brought to volume in a 50 mL volumetric flask for later use.
   2.4.4 The substrate protein FP1-SNAP25(17aa)-FP2 was thawed on ice.
   2.4.5 The microplate reader and its control computer were turned on, and the temperature in the reaction chamber was set to 37°C.
   2.4.6 Each enzymatic digestion system had a total volume of 50 µL. This experiment was conducted to validate the enzymatic activity of five single digestion Jianmeipeptide variants and to optimize the Jianmeipeptide-150 enzymatic digestion system.
   2.4.7 The reaction system was prepared in a 1.5 mL EP tube: In the system, the substrate protein FP1-SNAP25(17aa)-FP2 was at a concentration of 800 µg/mL, Jianmeipeptide-150 was at a concentration of 140 µg/mL, and the remaining volume was supplemented with the digestion buffer to a total of 50 µL. In the KLK5 digestion group, the substrate protein was at a concentration of 800 µg/mL, no Jianmeipeptide-150 was added, and only KLK5 protease was added at a concentration of 1.4 µg/mL (consistent with the concentration of KLK5 protease in the double digestion product). Two blank controls containing only FP1-SNAP25(17aa)-FP2 but no Jianmeipeptide-150 were set up.
   2.4.8 After mixing, the reaction system was added to a 384-well microplate, which was shaken in the microplate reader for 2 minutes.
   2.4.9 After shaking, the reaction program was adjusted to record readings every 2 minutes for 3 hours. The reading settings were as follows: excitation at 433 nm, emission at 476 nm; excitation at 433 nm, emission at 526 nm. Both excitation wavelengths were measured.
   2.4.10 After 3 hours of reading, the microplate was removed, and 3 µL of 0.25% Trypsin was added to each sample well to terminate the reaction. After addition of 0.25% Trypsin, the microplate was placed back into the microplate reader to start reading, with readings recorded per minute for half an hour. The reading settings were as follows: excitation at 433 nm, emission at 476 nm; excitation at 433 nm, emission at 526 nm. Both excitation wavelengths were measured.
2.5 Cleanup
   2.5.1 Data were completely recorded.
   2.5.2 Waste disposal: All waste was disposed into a chemical waste container.

As shown in FIG. 8, in a enzymatic digestion system containing Tween 80, the double digestion botulinum protein exhibited a significant cleavage effect on the substrate, while the single digestion botulinum proteins FA, FB, FC, FD, and HD (with only one first enzyme cleavage site cleaved by KLK5) showed no cleavage effect on the substrate (*i.e.,* subjected only to TEV protease digestion; the intermediate two cleavage sites were not cleaved, and none were active), and KLK5 showed essentially no enzymatic activity in the enzymatic digestion system containing Tween 80.

Example 6 demonstrates that the single-chain polypeptide provided by the present disclosure, due to the presence of the first enzyme cleavage site, requires cleavage by a highly specific first protease to be highly activated.

### Example 7: Experiment on products prepared by double enzymatic digestion

### (1) Efficacy test

### A. Experimental procedure for injection group

The modified target plasmid obtained according to Example 1 was used to prepare a single-chain polypeptide (obtained after elution and purification at pH 8.0) following the methods described in Examples 1 to 3. The protein obtained by double enzymatic digestion according to Example 4 was subjected to the following experiment.

Female CD-1 mice aged approximately 3 weeks (13 to 15 g) were purchased for the test. The mice were first transferred to an animal room for a 3-day acclimation period. After acclimation, the mice were subjected to an injection experiment. The stock protein solution was then diluted with phosphate-buffered saline to a concentration of 1 to 0.01 ng/mL to prepare an injection formulation, which was used for the injection experiment. Each mouse was firmly restrained by hand, the left hind limb was fixed with fingers, and the gastrocnemius muscle was located. 50 µL of the injection formulation was injected into the gastrocnemius muscle. In parallel, three mice were injected. After injection, the mice were observed for their condition. If normal, the mice were returned to their cages. The degree of syndactyly between the left and right hind limbs of the mice was observed and recorded daily.

### B. Experimental procedure for topical group

The single-chain polypeptide prepared according to Examples 1 to 3 and the protein obtained by double enzymatic digestion according to Example 4 were mixed with oleic acid, propylene glycol, Tween 80 to prepare a topical formulation (for the detailed preparation method for the topical formulation, refer to Chinese Patent Application No. 202310178312.0). The following experiment was conducted.

Female CD-1 mice aged approximately 3 weeks (13 to 15 g) were purchased for the test. The mice were first transferred to an animal room for a 3-day acclimation period. After acclimation, the topical formulation was tested on the mice. Following preparation of the topical formulation, a topical application test was conducted. The mice were first anesthetized by intraperitoneal injection of tribromoethanol, and the hair on the left hind limb was removed using a pet clipper to expose the gastrocnemius muscle. The topical formulation was then applied to the exposed area, with 200 µL per mouse (100 µL applied first to the gastrocnemius muscle, allowed to air dry, followed by another 100 µL). In parallel, three mice were used for each formulation. After application, the mice were observed for their condition and returned to their cages once the formulation had dried. The degree of syndactyly between the left and right hind limbs of the mice was observed and recorded daily, and the formulation was reapplied at the same initial dose.

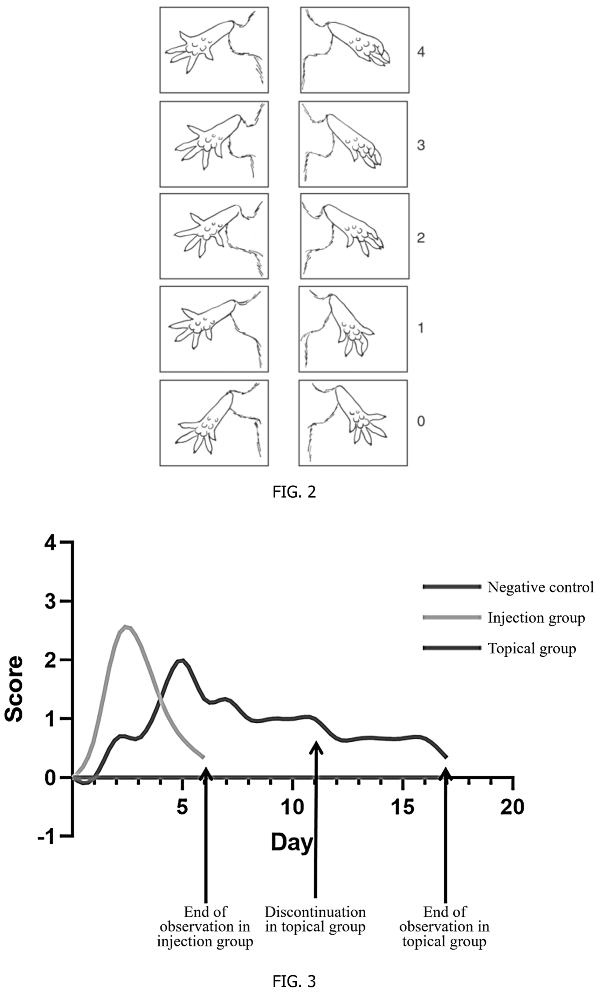

Referring to FIG. 2, the test effects of the two groups of samples were scored, in which the left side shows a reference without treatment, and the right side shows changes after treatment. Scores were assigned as 0, 1, 2, 3, 4, and 5, totaling five levels. The higher the score, the better the effect.

The experimental results are shown in FIG. 3, where the injection group used a protein concentration of 0.3 ng/mL and the topical group used a protein concentration of 10 µg/mL.

Based on the experimental results:
(1) A single administration in the injection group showed the strongest efficacy on day 3 after administration, and the efficacy nearly disappeared by day 5 after administration;
(2) Continuous administration in the topical group for 11 days showed the strongest efficacy on day 5 after administration, and after discontinuation, the efficacy lasted significantly longer than that in the injection group.

### (2) Acute oral toxicity test - gavage experiment

Female CD-1 mice aged approximately 3 weeks (13 to 15 g) were purchased for the test. The mice were first transferred to an animal room for a 3-day acclimation period. After acclimation, the mice were subjected to a gavage experiment. Subsequently, the mice were administered with the aforementioned injection formulation and topical formulation via oral gavage.

Specifically, each mouse was firmly restrained by hand, a 1 mL syringe was used to draw 300 µL of the injection or topical formulation, and an 8-gauge gavage needle was used for the gavage procedure. In parallel, three mice were administered via oral gavage. After gavage, the mice were observed for their condition. If normal, the mice were returned to their cages. The behavioral status of the mice was observed daily, and the body weight of the mice was recorded.

Experimental results: No deaths occurred in either the injection group or the topical group, and the body weight gain trend was consistent with that of the control group.

### Example 8: Experiment without enzymatic digestion

(1) According to the method described in Example 1, a target plasmid containing a nucleotide sequence encoding a protein structure of formula I was constructed:
   LC-D-HC (formula II, connected from the N-terminus to the C-terminus);

In the formula, D represents the intermediate amino acid sequence region that contains the first enzyme cleavage site corresponding to different first proteases, including KLK5, KLK7, SASPase, and Caspase-14; LC represents the first domain, whose amino acid sequence is shown in SEQ ID NO: 10 in this example; HC represents the second domain, whose amino acid sequence is shown in SEQ ID NO: 17 in this example.

The specific structures are shown in Table 3.

| Table 3 | |
|---|---|
| Protein ID | Structure |
| FeHD | L-furin(RRKR)-GG-KLK5-H |
| FfHDB | L-furin(RTRR)-GG-KLK5-H |
| FfHDS | L-furin(RTKR)-GG-KLK5-H |
| HD | L-QTQS-SGG-KLK5-H |
| KCK5S (KLK5S) | L-QTQS-GG-KLK5-H |
| m2 | L-GI-furin(RRKR)-SVSH-KLK5-H |
| m4 | L-furin(RRKR)-SVSH-KLK5-H |
| m6 | L-furin(RTKR)-SVS-KLK5-H |
| NTF17 | L-furin(RTRR)-SVS-KLK5-H |
| NTF23 | L-furin(RTRR)-GG-H |
| NTF24 | L-furin(RTKR)-GG-H |
| NTF25 | L-furin(RRSV)-GG-H |
| NTF26 | L-furin(RRKR)-GG-H |
| NTF27 | L-QTQS-KLK7-H |
| NTF28 | L-QTQS-SASPase-H |
| NTF29 | L-QTQS-Caspase-14a-H |
| NTF32K | L-furin(RTKR)-GG-KLK5-H |
| Control 1 | L-QTQS-TEV-H |
| Control 2 | L-QTQS-GG-H |

The amplification products (DNA templates) corresponding to the structures listed in Table 3 were expressed and purified according to the methods described in Examples 2 to 3 to obtain various proteins.

Electrophoresis results showed that the obtained proteins exhibited no significant cleavage, demonstrating that these proteins were not easily activated *in vitro* without exposure to specific first proteases.

### (1) Topical application test

### 1. Materials and animals

1.1 Sample information: Each sample protein was formulated into preparations with a concentration of 10 µg/mL. All samples were stored protected from light at 2°C to 8°C.

1.2 Animal strain: CD1 mice, sourced from Zhejiang Vital River Laboratory Animal Technology Co., Ltd.; a total of 24 female mice, weighing 18 to 22 g, SPF-grade; date of introduction: 20230407; laboratory animal production license No.: SCXK (Zhejiang) 2019-0001; laboratory animal quality certificate No.: 20220714Abzz0619000520; laboratory animal use license No.: SYXK (Shanghai) 2022-0015.

1.3 Feed source: Shanghai Puluteng Biotechnology Co., Ltd.; SPF-grade maintenance feed for mice; production license No.: Shanghai Feed Certificate (2018) 04001.

### 2. Methods

2.1 Detection method: The Digit Abduction Score (DAS) was used to assess the degree of denervation in the hind limbs of mice, with scoring performed according to the criteria shown in FIG. 2.

2.2 Experimental method: The prepared sample proteins were brought to room temperature. Mice were randomly grouped and anesthetized by intraperitoneal injection of 2.5% tribromoethanol. After anesthesia, the hair on the skin over the gastrocnemius muscle was shaved and wiped with 10% ethanol. The test sample solutions were topically applied to the gastrocnemius muscle of CD-1 mice, with 0.2 mL per mouse and three mice per sample, continuously for 7 days. After application, the mice were continuously observed, and mortality and syndactyly results were recorded daily. The strength of the effect was evaluated based on the syndactyly scoring criteria.

Conclusion: The results confirmed that the proteins listed in Table 3, when topically applied, exhibited an earlier onset of effect and a longer duration of effect compared to Control 1 and Control 2.

(2) LD50

### 1. Materials and animals

1.1 Sample information: FfHDS (Jianmeipeptide-150 (Ff), Km-JMT-0150), a colorless and transparent liquid, stored protected from light at 2°C to 8°C.

1.2 Animal strain: CD1 mice, sourced from Zhejiang Vital River Laboratory Animal Technology Co., Ltd.; a total of 25 female mice, weighing 18 to 22 g, SPF-grade; date of introduction: 20230217; laboratory animal production license No.: SCXK (Zhejiang) 2019-0001; laboratory animal quality certificate No.: 20220714Abzz0619000520; laboratory animal use license No.: SYXK (Shanghai) 2022-0015.

1.3 Feed source: Shanghai Puluteng Biotechnology Co., Ltd.; SPF-grade maintenance feed for mice; production license No.: Shanghai Feed Certificate (2018) 04001.

### 2. Methods

2.1 Detection method: Chinese Pharmacopoeia (2020 Edition), Part IV, General Rule 3533 - Potency Assay of Botulinum Toxin Type A.

2.2 Experimental method: Jianmeipeptide-150 (Ff) with an initial concentration of 750 µg/mL was serially diluted with 0.9% sodium chloride solution at equal ratios to obtain six concentrations: 2 ng/mL, 1 ng/mL, 0.5 ng/mL, 0.25 ng/mL, 0.125 ng/mL, and 0.0625 ng/mL, corresponding to doses of 38.46 ng/kg, 19.23 ng/kg, 9.62 ng/kg, 4.81 ng/kg, 2.40 ng/kg, and 1.20 ng/kg, respectively. The diluted test sample solutions were injected into female Kunming mice aged 26 to 30 days (SPF-grade). A total of 6 concentration groups were injected intraperitoneally at a dose of 0.5 mL per mouse, with 5 mice in the highest concentration group and 4 mice in each of the remaining groups. The groups were labeled as 1, 2, 3, 4, 5, and 6, respectively. After injection, the mice were continuously observed for five days, and mortality was recorded daily. Based on the dose-response curve of 4-day survival rate, the biological potency of the test sample was calculated.

### 3. Results

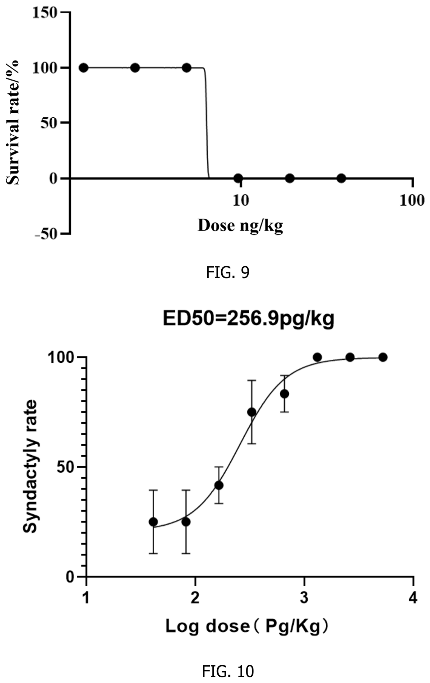

Data processing was performed using Excel, and graphical analysis was conducted using GraphPad (see FIG. 9). The calculation was based on the modified Karber method, using the following formula: *LD*₅₀ = log⁻¹ [Xₘ - i (Σp - 0.5)]:

Xₘ represents the logarithm of the dose in the maximum dose group; i represents the logarithm of the ratio of high dose to low dose between two adjacent groups (the logarithmic difference between two adjacent groups); p represents the mortality rate in each group; Σp represents the sum of mortality rates across all groups.

Calculation results: LD50 = 6.8 ng/kg. The potency unit was 0.136 ng/U (calculated based on 20 g per mouse) (*i.e.,* LD50).

The obtained potency was significantly lower than that of natural botulinum toxin (Rossetto, O.; Montecucco, C. Tables of Toxicity of Botulinum and Tetanus Neurotoxins. Toxins 2019, 11, 686. [CrossRef] [PubMed]).

Botulinum neurotoxins ("BoNT", seven immunologically distinct subtypes, A to G) and tetanus neurotoxins ("TeNT") are known to be the most toxic substances by weight, with LD50 values ranging from 0.5 to 2.5 ng/kg when administered intravenously or intramuscularly (National Institute of Occupational Safety and Health, "Registry of Toxic Effects of Chemical Substances (R-TECS)," Cincinnati, Ohio: National Institute of Occupational Safety and Health (1996)).

The toxicity of wild-type botulinum toxin type A (natural botulinum toxin type A) was approximately 20 times higher than that of the polypeptide designed in the present disclosure.

### (3) ED50

**1. Experimental objective: To determine ED50**
**2. Experimental procedure**
   2.1 Reagents and consumables
      2.1.1 0.9% sodium chloride solution
      2.1.2 Km-JMT-0150 (FfHDS, Jianmeipeptide-150 (Ff)), 750 µg/mL
      2.1.3 Female SPF-grade CD-1 mice aged 26 to 30 days (Zhejiang Vital River Laboratory Animal Technology Co., Ltd.)
      2.1.4 1 mL medical syringe
      2.1.5 Purified water
      2.1.6 50 mL centrifuge tube
      2.1.7 1 mL pipette tip
      2.1.8 200 µL pipette tip
      2.1.9 10 µL pipette tip
      2.1.10 1.5 mL EP tube
      2.1.11 Feed for mice
      2.1.12 Corn cob bedding for mice
      2.1.13 Purified drinking water for mice
   2.2 Equipment
      2.2.1 Clean bench
      2.2.2 1 mL pipette
      2.2.3 200 µL pipette
      2.2.4 10 µL pipette
   2.3 Experimental procedure for ED50 of Km-JMT-0150 in mice
      2.3.1 Pre-experiment preparation
      2.3.1.1 Disposable medical syringes, CD-1 mice of the appropriate age, and Km-JMT-0150 at different concentration gradients were prepared.
      2.3.1.2 The clean bench was sterilized with ultraviolet light for 30 minutes.
      2.3.1.3 The experimental information was recorded in the "Animal Experiment Record".
      2.3.2 Test sample preparation
         Preparation of Km-JMT-0150 at different concentration gradients: Km-JMT-0150 was serially diluted with 0.9% sodium chloride solution at equal ratios to obtain eight concentrations: 1.68 ng/mL, 0.84 ng/mL, 0.42 ng/mL, 0.21 ng/mL, 0.105 ng/mL, 0.0525 ng/mL, 0.02625 ng/mL, and 0.013125 ng/mL. The groups were labeled as 1, 2, 3, 4, 5, 6, 7, and 8, respectively.
      2.3.3 Experimental method
      2.3.3.1 Twenty-four female SPF-grade CD-1 mice aged 4 weeks were selected and weighed, and their survival status was observed for subsequent gastrocnemius muscle injection.
      2.3.3.2 Mouse handling: Each mouse was lifted by the tail with the right hand and placed on the cage or bench, and then gently pulled backward. As the mouse moved forward, the skin of ears and neck was grasped with the left thumb and index finger. The mouse was placed in the left palm, with the hind limbs extended. The tail was pressed with the ring finger and the hind limbs were pressed with the little finger, ensuring that the mouse was firmly restrained.
      2.3.3.3 After the mouse was firmly restrained, diluted Km-JMT-0150 at eight different concentration gradients was administered via gastrocnemius muscle injection into CD-1 mice. Three mice were injected per gradient, with an injection volume of 60 µL per mouse.
      2.3.3.4 After injection, the mice were continuously observed for five days, and the degree of syndactyly was recorded daily. Based on the dose-response curve of syndactyly rate on the day of most severe manifestation, the ED50 of the test sample was calculated.
      2.3.4 Precautions
      2.3.4.1 When preparing Km-JMT-0150 at different concentration gradients, thorough mixing was required before performing serial dilutions.
      2.3.4.2 During gastrocnemius muscle injection, the syringe needle was rapidly inserted into the gastrocnemius muscle at a 90° angle to a depth of 0.3 cm for injection.
      2.3.4.3 After injection, to prevent leakage, the needle was slightly rotated before being slowly withdrawn.
   2.4 Cleanup
      2.4.1 The injected mice were returned to their cages. Used needles were disposed of in a sharps container, and other waste was placed in a biohazard waste bin.
      2.4.2 The surface of the clean bench was wiped with 75% alcohol cotton balls until clean.
**3. Results and analysis**

FIG. 10 shows the ED50 dose-response curve derived by plotting the degree of syndactyly (using the scoring criteria described above) against the logarithm of the dose via gastrocnemius muscle injection in mice. Based on the dose-response curve, the ED50 (dose corresponding to a score of 2) of Km-JMT-0150 was calculated to be 256.9 pg/kg = 0.2569 ng/kg.

Observation of the mice revealed that syndactyly was most severe at 48 hours after gastrocnemius muscle injection of Km-JMT-0150. The more severe the syndactyly, the slower the recovery; conversely, the milder the syndactyly, the faster the recovery.

The safety margin (LD50/ED50) of Km-JMT-0150 was calculated to be approximately 27.

By comparison, the safety margin of wild-type BoNT A1 was 13.

Thus, the present disclosure provides a significantly reduced toxicity and a better safety margin.

The sequences mentioned above are summarized in Table 4.

| Table 4 | | |
|---|---|---|
| Name | Sequence No. | Amino acid sequence or nucleotide sequence |
| First enzyme cleavage site (cleavable by KLK5) | SEQ ID NO: 1 | YRSRIV |
| First enzyme cleavage site (cleavable by KLK7) | SEQ ID NO: 2 | KHLYSN |
| First enzyme cleavage site (cleavable by SASPase) | SEQ ID NO: 3 | VSQVYPIVQ |
| First enzyme cleavage site (cleavable by Caspase-14) | SEQ ID NO: 4 | WEHDGIAE |
| Second enzyme cleavage site | SEQ ID NO: 5 | DDDK |
| Second enzyme cleavage site (TEV cleavage) | SEQ ID NO: 6 | ENLYFQS |
| Second enzyme cleavage site | SEQ ID NO: 7 | HY |
| Second enzyme cleavage site | SEQ ID NO: 8 | YH |
| Second enzyme cleavage site | SEQ ID NO: 9 | LEVLFQGP |
| First structure (A light chain) | SEQ ID NO: 10 | MPFVNKQFNYKDPVNGVDIAYIKIPNVGQMQPVKAFKIHNKIW VIPERDTFTNPEEGDLNPPPEAKQVPVSYYDSTYLSTDNEKDNY LKGVTKLFERIYSTDLGRMLLTSIVRGIPFWGGSTIDTELKVIDT NCINVIQPDGSYRSEELNLVIIGPSADIIQFECKSFGHEVLNLTRN GYGSTQYIRFSPDFTFGFEESLEVDTNPLLGAGKFATDPAVTLAH ELIHAGHRLYGIAINPNRVFKVNTNAYYEMSGLEVSFEELRTFGG HDAKFIDSLQENEFRLYYYNKFKDIASTLNKAKSIVGTTASLQYM KNVFKEKYLLSEDTSGKFSVDKLKFDKLYKMLTEIYTEDNFVKFF KVLNRKTYLNFDKAVFKINIVPKVNYTIYDGFNLRNTNLAANFN GQNTEINNMNFTKLKNFTGLFEFYKLLCVRGIITS |
| First structure (B light chain) | SEQ ID NO: 11 | MPVTINNFNYNDPIDNNNIIMMEPPFARGTGRYYKAFKITDRIW IIPERYTFGYKPEDFNKSSGIFNRDVCEYYDPDYLNTNDKKNIFL QTMIKLFNRIKSKPLGEKLLEMIINGIPYLGDRRVPLEEFNTNIAS VTVNKLISNPGEVERKKGIFANLIIFGPGPVLNENETIDIGIQNHF ASREGFGGIMQMKFCPEYVSVFNNVQENKGASIFNRRGYFSDP ALILMHELIHVLHGLYGIKVDDLPIVPNEKKFFMQSTDAIQAEEL YTFGGQDPSIITPSTDKSIYDKVLQNFRGIVDRLNKVLVCISDPN ININIYKNKFKDKYKFVEDSEGKYSIDVESFDKLYKSLMFGFTET NIAENYKIKTRASYFSDSLPPVKIKNLLDNEIYTIEEGFNISDKNM EKEYRGQNKAINKQAYEEISKEHLAVYKIQMCKSVR |
| First structure (C light chain) | SEQ ID NO: 12 | MPITINNFNYSDPVDNKNILYLDTHLNTLANEPEKAFRITGNIW VIPDRFSRNSNPNLNKPPRVTSPKSGYYDPNYLSTDSDKDTFLK EIIKLFKRINSREIGEELIYRLSTDIPFPGNNNTPINTFDFDVDFN SVDVKTRQGNNWVKTGSINPSVIITGPRENIIDPETSTFKLTNN TFAAQEGFGALSIISISPRFMLTYSNATNDVGEGRFSKSEFCMDP ILILMHELNHAMHNLYGIAIPNDQTISSVTSNIFYSQYNVKLEYA EIYAFGGPTIDLIPKSARKYFEEKALDYYRSIAKRLNSITTANPSS FNKYIGEYKQKLIRKYRFVVESSGEVTVNRNKFVELYNELTQIFT EFNYAKIYNVQNRKIYLSNVYTPVTANILDDNVYDIQNGFNIPKS NLNVLFMGQNLSRNPALRKVNPENMLYLFTKFCHKAIDGR |
| First structure (D light chain) | SEQ ID NO: 13 | MTWPVKDFNYSDPVNDNDILYLRIPQNKLITTPVKAFMITQNIW VIPERFSSDTNPSLSKPPRPTSKYQSYYDPSYLSTDEQKDTFLKG IIKLFKRINERDIGKKLINYLVVGSPFMGDSSTPEDTFDFTRHTT NIAVEKFENGSWKVTNIITPSVLIFGPLPNILDYTASLTLQGQQS NPSFEGFGTLSILKVAPEFLLTFSDVTSNQSSAVLGKSIFCMDPVI ALMHELTHSLHQLYGINIPSDKRIRPQVSEGFFSQDGPNVQFEE LYTFGGLDVEIIPQIERSQLREKALGHYKDIAKRLNNINKTIPSS WISNIDKYKKIFSEKYNFDKDNTGNFVVNIDKFNSLYSDLTNVM SEVVYSSQYNVKNRTHYFSRHYLPVFANILDDNIYTIRDGFNLT NKGFNIENSGQNIERNPALQKLSSESVVDLFTKVCLRLTKNSR |
| First structure (E light chain) | SEQ ID NO: 14 | MPTINSFNYNDPVNNRTILYIKPGGCQQFYKSFNIMKNIWIIPER NVIGTIPQDFLPPTSLKNGDSSYYDPNYLQSDQEKDKFLKIVTKI FNRINDNLSGRILLEELSKANPYLGNDNTPDGDFIINDASAVPIQ FSNGSQSILLPNVIIMGAEPDLFETNSSNISLRNNYMPSNHGFG SIAIVTFSPEYSFRFKDNSMNEFIQDPALTLMHELIHSLHGLYGA KGITTKYTITQKQNPLITNIRGTNIEEFLTFGGTDLNIITSAQSND IYTNLLADYKKIASKLSKVQVSNPLLNPYKDVFEAKYGLDKDASG IYSVNINKFNDIFKKLYSFTEFDLATKFQVKCRQTYIGQYKYFKLS NLLNDSIYNISEGYNINNLKVNFRGQNANLNPRIITPITGRGLVK KIIRFCKNIVSVKGIR |
| First structure (F light chain) | SEQ ID NO: 15 | MPVAINSFNYNDPVNDDTILYMQIPYEEKSKKYYKAFEIMRNVW IIPERNTIGTNPSDFDPPASLKNGSSAYYDPNYLTTDAEKDRYLK TTIKLFKRINSNPAGKVLLQEISYAKPYLGNDHTPIDEFSPVTRTT SVNIKLSTNVESSMLLNLLVLGAGPDIFESCCYPVRKLIDPDVVY DPSNYGFGSINIVTFSPEYEYTFNDISGGHNSSTESFIADPAISLA HELIHALHGLYGARGVTYEETIEVKQAPLMIAEKPIRLEEFLTFGG QDLNIITSAMKEKIYNNLLANYEKIATRLSEVNSAPPEYDINEYK DYFQWKYGLDKNADGSYTVNENKFNEIYKKLYSFTESDLANKF KVKCRNTYFIKYEFLKVPNLLDDDIYTVSEGFNIGNLAVNNRGQ SIKLNPKIIDSIPDKGLVEKIVKFCKSVIPRKGTK |
| First structure (G light chain) | SEQ ID NO: 16 | MPVNIKXFNYNDPINNDDIIMMEPFNDPGPGTYYKAFRIIDRIW IVPERFTYGFQPDQFNASTGVFSKDVYEYYDPTYLKTDAEKDKF LKTMIKLFNRINSKPSGQRLLDMIVDAIPYLGNASTPPDKFAANV ANVSINKKIIQPGAEDQIKGLMTNLIIFGPGPVLSDNFTDSMIM NGHSPISEGFGARMMIRFCPSCLNVFNNVQENKDTSIFSRRAYF ADPALTLMHELIHVLHGLYGIKISNLPITPNTKEFFMQHSDPVQA EELYTFGGHDPSVISPSTDMNIYNKALQNFQDIANRLNIVSSAQ GSGIDISLYKQIYKNKYDFVEDPNGKYSVDKDKFDKLYKALMFG FTETNLAGEYGIKTRYSYFSEYLPPIKTEKLLDNTIYTQNEGFNIA SKNLKTEFNGQNKAVNKEAYEEISLEHLVIYRIAMCKPVMYKNT GK |
| Second structure (A heavy chain) | SEQ ID NO: 17 | ALNDLCIKVNNWDLFFSPSEDNFTNDLNKGEEITSDTNIEAAEE NISLDLIQQYYLTFNFDNEPENISIENLSSDIIGQLELMPNIERFP NGKKYELDKYTMFHYLRAQEFEHGKSRIALTNSVNEALLNPSRV YTFFSSDYVKKVNKATEAAMFLGWVEQLVYDFTDETSEVSTTD KIADITIIIPYIGPALNIGNMLYKDDFVGALIFSGAVILLEFIPEIAI PVLGTFALVSYIANKVLTVQTIDNALSKRNEKWDEVYKYIVTNW LAKVNTQIDLIRKKMKEALENQAEATKAIINYQYNQYTEEEKNN INFNIDDLSSKLNESINKAMININKFLNQCSVSYLMNSMIPYGVK RLEDFDASLKDALLKYIYDNRGTLIGQVDRLKDKVNNTLSTDIP FQLSKYVDNQRLLSTFTEYINNIINTSILNLRYESNHLIDLSRYAS KINIGSKVNFDPIDKNQIQLFNLESSKIEVILKNAIVYNSMYENFS TSFWIRIPKYFNSISLNNEYTIINCMENNSGWKVSLNYGEIIWTL QDTQEIKQRVVFKYSQMINISDYINRWIFVTITNNRLNNSKIYI NGRLIDQKPISNLGNIHASNNIMFKLDGCRDTHRYIWIKYFNLF DKELNEKEIKDLYDNQSNSGILKDFWGDYLQYDKPYYMLNLYD PNKYVDVNNVGIRGYMYLKGPRGSVMTTNIYLNSSLYRGTKFII KKYASGNKDNIVRNNDRVYINVVVKNKEYRLATNASQAGVEKIL SALEIPDVGNLSQVVVMKSKNDQGITNKCKMNLQDNNGNDIG FIGFHQFNNIAKLVASNWYNRQIERSSRTLGCSWEFIPVDDGW GERPL |
| Second structure (B heavy chain) | SEQ ID NO: 18 | APGICIDVDNEDLFFIADKNSFSDDLSKNERIEYDTQSNYIENRS SINELILDTNLISKIELPSENTESLTDFNVDVPVYEKQPAIKKIFTD ENTIFQYLYSQTFPLDIRDISLTSSFDDALLFSNKVYSFFSMDYIK TANKVVEAGLFAGWVKQIVDDFVIEANKSSTMDKIADISLIVPYI GLALNVGNETAKGNFENAFEIAGASILLEFIPELLIPVVGAFLLES YIDNKNKIIKTIDNALTKRDEKWIDMYGLIVAQWLSTVNTQFYT IKEGMYKALNYQAQALEEIIKYKYNIYSEKEKSNINIDFNDINSKL NEGINQAIDNINDFINECSVSYLMKKMIPLAVEKLLDFDNTLKKN LLNYIDENKLYLIGSAEYEKSKVDKYLKTIIPFDLSMYTNNTILIEI FNKYNSEILNNIILNLRYRDNNLIDLSGYGANVEVYDGVKLNDK NQFKLTSSTNSEIRVTQNQNIIFNSMFLDFSVSFWIRIPKYKND GIQNYIHNEYTIINCIKNNSGWKISIRGNRIIWTLTDINGKTKSV FFEYSIREDISDYINRWFFVTITNNSDNAKIYINGKLESNIDIKNI GEVIANGEIIFKLDGDIDRTQFIWMKYFSIFNTELSQSNIKEIYKI QSYSEYLKDFWGNPLMYNKEYYMFNAGNKNSYIKLKKDSSVGE ILTRSKYNQNSNYINYRNLYIGEKFIIRRKSNSQSINDDIVRKED YIYLDFFNSNREWRVYAYKDFKEEEKKLFLANIYDSNEFYKTIQI KEYDEQPTYSCQLLFKKDEESTDEIGLIGIHRFYESGIVLKDYKN YFCISKWYLKEVKRKPYNPNLGCNWQFIPKDEGWIE |
| Second structure (C heavy chain) | SEQ ID NO: 19 | SLYNKTLDCRELLVKNTDLPFIGDISDVKTDIFLRKDINEETEVIY YPDNVSVDQVILSKNTSEHGQLDLLYPSIDSESEILPGENQVFYD NRTQNVDYLNSYYYLESQKLSDNVEDFTFTRSIEEALDNSAKVY TYFPTLANKVNAGVQGGLFLMWANDVVEDFTTNILRKDTLDKI SDVSAIIPYIGPALNISNSVRRGNFTEAFAVTGVTILLEAFPEFTIP ALGAFVIYSKVQERNEIIKTIDNCLEQRIKRWKDSYEWMMGTW LSRIITQFNNISYQMYDSLNYQAGAIKAKIDLEYKKYSGSDKENI KSQVENLKNSLDVKISEAMNNINKFIRECSVTYLFKNMLPKVIDE LNEFDRNTKAKLINLIDSHNIILVGEVDKLKAKVNNSFQNTIPFN IFSYTNNSLLKDIINEYFNNINDSKILSLQNRKNTLVDTSGYNAE VSEEGDVQLNPIFPFDFKLGSSGEDRGKVIVTQNENIVYNSMYE SFSISFWIRINKWVSNLPGYTIIDSVKNNSGWSIGIISNFLVFTL KQNEDSEQSINFSYDISNNAPGYNKWFFVTVTNNMMGNMKIYI NGKLIDTIKVKELTGINFSKTITFEINKIPDTGLITSDSDNINMWI RDFYIFAKELDGKDINILFNSLQYTNVVKDYWGNDLRYNKEYY MVNIDYLNRYMYANSRQIVFNTRRNNNDFNEGYKIIIKRIRGNT NDTRVRGGDILYFDMTINNKAYNLFMKNETMYADNHSTEDIYA IGLREQTKDINDNIIFQIQPMNNTYYYASQIFKSNFNGENISGIC SIGTYRFRLGGDWYRHNYLVPTVKQGNYASLLESTSTHWGFVP VSE |
| Second structure (D heavy chain) | SEQ ID NO: 20 | DDSTCIKVKNNRLPYVADKDSISQEIFENKIITDETNVQNYSDKF SLDESILDGQVPINPEIVDPLLPNVNMEPLNLPGEEIVFYDDITK YVDYLNSYYYLESQKLSNNVENITLTTSVEEALGYSNKIYTFLPSL AEKVNKGVQAGLFLNWANEVVEDFTTNIMKKDTLDKISDVSVII PYIGPALNIGNSALRGNFNQAFATAGVAFLLEGFPEFTIPALGVF TFYSSIQEREKIIKTIENCLEQRVKRWKDSYQWMVSNWLSRITT QFNHINYQMYDSLSYQADAIKAKIDLEYKKYSGSDKENIKSQVE NLKNSLDVKISEAMNNINKFIRECSVTYLFKNMLPKVIDELNKFD LRTKTELINLIDSHNIILVGEVDRLKAKVNESFENTMPFNIFSYTN NSLLKDIINEYFNSINDSKILSLQNKKNALVDTSGYNAEVRVGDN VQLNTIYTNDFKLSSSGDKIIVNLNNNILYSAIYENSSVSFWIKIS KDLTNSHNEYTIINSIEQNSGWKLCIRNGNIEWILQDVNRKYKS LIFDYSESLSHTGYTNKWFFVTITNNIMGYMKLYINGELKQSQK IEDLDEVKLDKTIVFGIDENIDENQMLWIRDFNIFSKELSNEDIN IVYEGQILRNVIKDYWGNPLKFDTEYYIINDNYIDRYIAPESNVL VLVQYPDRSKLYTGNPITIKSVSDKNPYSRILNGDNIILHMLYNS RKYMIIRDTDTIYATQGGECSQNCVYALKLQSNLGNYGIGIFSIK NIVSKNKYCSQIFSSFRENTMLLADIYKPWRFSFKNAYTPVAVT NYETKLLSTSSFWKFISRDPGWVE |
| Second structure (E heavy chain) | SEQ ID NO: 21 | KSICIEINNGELFFVASENSYNDDNINTPKEIDDTVTSNNNYEN DLDQVILNFNSESAPGLSDEKLNLTIQNDAYIPKYDSNGTSDIEQ HDVNELNVFFYLDAQKVPEGENNVNLTSSIDTALLEQPKIYTFFS SEFINNVNKPVQAALFVGWIQQVLVDFTTEANQKSTVDKIADIS IVVPYIGLALNIGNEAQKGNFKDALELLGAGILLEFEPELLIPTILV FTIKSFLGSSDNKNKVIKAINNALKERDEKWKEVYSFIVSNWMT KINTQFNKRKEQMYQALQNQVNALKAIIESKYNSYTLEEKNELT NKYDIEQIENELNQKVSIAMNNIDRFLTESSISYLMKLINEVKIN KLREYDENVKTYLLDYIIKHGSILGESQQELNSMVIDTLNNSIPF KLSSYTDDKILISYFNKFFKRIKSSSVLNMRYKNDKYVDTSGYDS NININGDVYKYPTNKNQFGIYNDKLSEVNISQNDYIIYDNKYKN FSISFWVRIPNYDNKIVNVNNEYTIINCMRDNNSGWKVSLNHN EIIWTLQDNSGINQKLAFNYGNANGISDYINKWIFVTITNDRLG DSKLYINGNLIDKKSILNLGNIHVSDNILFKIVNCSYTRYIGIRYF NIFDKELDETEIQTLYNNEPNANILKDFWGNYLLYDKEYYLLNVL KPNNFINRRTDSTLSINNIRSTILLANRLYSGIKVKIQRVNNSST NDNLVRKNDQVYINFVASKTHLLPLYADTATTNKEKTIKISSSGN RFNQVVVMNSVGNCTMNFKNNNGNNIGLLGFKADTVVASTWY YTHMRDNTNSNGFFWNFISEEHGWQEK |
| Second structure (F heavy chain) | SEQ ID NO: 22 | APPRLCIRVNNSELFFVASESSYNENDINTPKEIDDTTNLNNNYR NNLDEVILDYNSQTIPQISNRTLNTLVQDNSYVPRYDSNGTSEI EEYDVVDFNVFFYLHAQKVPEGETNISLTSSIDTALLEESKDIFFS SEFIDTINKPVNAALFIDWISKVIRDFTTEATQKSTVDKIADISLI VPYVGLALNIIIEAEKGNFEEAFELLGVGILLEFVPELTIPVILVFTI KSYIDSYENKNKAIKAINNSLIEREAKWKEIYSWIVSNWLTRINT QFNKRKEQMYQALQNQVDAIKTAIEYKYNNYTSDEKNRLESEY NINNIEEELNKKVSLAMKNIERFMTESSISYLMKLINEAKVGKLK KYDNHVKSDLLNYILDHRSILGEQTNELSDLVTSTLNSSIPFELS SYTNDKILIIYFNRLYKKIKDSSILDMRYENNKFIDISGYGSNISI NGNVYIYSTNRNQFGIYNSRLSEVNIAQNNDIIYNSRYQNFSISF WVRIPKHYKPMNHNREYTIINCMGNNNSGWKISLRTVRDCEII WTLQDTSGNKENLIFRYEELNRISNYINKWIFVTITNNRLGNSR IYINGNLIVEKSISNLGDIHVSDNILFKIVGCDDETYVGIRYFKVF NTELDKTEIETLYSNEPDPSILKNYWGNYLLYNKKYYLFNLLRKD KYITLNSGILNINQQRGVTEGSVFLNYKLYEGVEVIIRKNGPIDIS NTDNFVRKNDLAYINVVDRGVEYRLYADTKSEKEKIIRTSNLND SLGQIIVMDSIGNNCTMNFQNNNGSNIGLLGFHSNNLVASSWY YNNIRRNTSSNGCFWSSISKENGWKE |
| Second structure (G heavy chain) | SEQ ID NO: 23 | SEQCIIVNNEDLFFIANKDSFSKDLAKAETIAYNTQNNTIENNFS IDQLILDNDLSSGIDLPNENTEPFTNFDDIDIPVYIKQSALKKIFV DGDSLFEYLHAQTFPSNIENLQLTNSLNDALRNNNKVYTFFSTN LVEKANTVVGASLFVNWVKGVIDDFTSESTQKSTIDKVSDVSIII PYIGPALNVGNETAKENFKNAFEIGGAAILMEFIPELIVPIVGFFT LESYVGNKGHIIMTISNALKKRDQKWTDMYGLIVSQWLSTVNT QFYTIKERMYNALNNQSQAIEKIIEDQYNRYSEEDKMNINIDFN DIDFKLNQSINLAINNIDDFINQCSISYLMNRMIPLAVKKLKDFD DNLKRDLLEYIDTNELYLLDEVNILKSKVNRHLKDSIPFDLSLYTK DTILIQVFNNYISNISSNAILSLSYRGGRLIDSSGYGATMNVGSD VIFNDIGNGQFKLNNSENSNITAHQSKFVVYDSMFDNFSINFW VRTPKYNNNDIQTYLQNEYTIISCIKNDSGWKVSIKGNRIIWTLI DVNAKSKSIFFEYSIKDNISDYINKWFSITITNDRLGNANIYING SLKKSEKILNLDRINSSNDIDFKLINCTDTTKFVWIKDFNIFGREL NATEVSSLYWIQSSTNTLKDFWGNPLRYDTQYYLFNQGMQNIY IKYFSKASMGETAPRTNFNNAAINYQNLYLGLRFIIKKASNSRNI NNDNIVREGDYIYLNIDNISDESYRVYVLVNSKEIQTQLFLAPIN DDPTFYDVLQIKKYYEKTTYNCQILCEKDTKTFGLFGIGKFVKDY GYVWDTYDNYFCISQWYLRRISENINKLRLGCNWQFIPVDEG WTE |
| SP | SEQ ID NO: 24 | MITETSSPFRSIFSHSGK |
| His tag | SEQ ID NO: 25 | HHHHHHHH |
| EGFP | SEQ ID NO: 26 | VSKGEELFTGVVPILVELDGDVNGHKFSVRGEGEGDATNGKLTL KFICTTGKLPVPWPTLVTTLTYGVQCFSRYPDHMKQHDFFKSAM PEGYVQERTISFKDDGTYKTRAEVKFEGDTLVNRIELKGIDFKE DGNILGHKLEYNFNSHNVYITADKQKNGIKANFKIRHNVEDGSV QLADHYQQNTPIGDGPVLLPDNHYLSTQSKLSKDPNEKRDHMV LLEFVTAAGITLGMDELYK |
| KLK5-F | SEQ ID NO: 27 | TACAGATCTAGAATCGTT GCTTTGAACGACTTGTGTATC |
| KLK5-R | SEQ ID NO: 28 | AACGATTCTAGATCTGTAAGACTTGGTCTTAGAGGTGATG |
| KLK7-F | SEQ ID NO: 29 | AAGCACTRGTACTCTAAC GCTTTGAACGACTTGTGTATC |
| KLK7-R | SEQ ID NO: 30 | GTTAGAGTACAAGTGCTTAGACTTGGTCTTAGAGGTGATG |
| SASPase-F | SEQ ID NO: 31 | GTTTCTCAAGTTTACCCAATCGTTCAA GCTTTGAACGACTTGTGTATC |
| SASPase-R | SEQ ID NO: 32 | TGGGTAAACTTGAGAAACAGACTTGGTCTTAGAGGTGATG |
| Caspase-14-F | SEQ ID NO: 33 | GGTGGTTGGGAACACGACGGTATCGCTTTC GCTTTGAACGACTTGTGTATC |
| Caspase-14-R | SEQ ID NO: 34 | GTGTTCCCAA CCACCAGACT TGGTCTTAGA GGTGATG |
| KLK5-2Q-F | SEQ ID NO: 35 | CTCAGACCCAGTCTGGTGGT TACAGATCTAGAATCGTT GCTTTGAACGAC |
| KLK5-2Q-R | SEQ ID NO: 36 | ACCAGACTGGGTCTGAGAGGTGATGATACCTCTAACACAC |
| KLK7-2Q-F | SEQ ID NO: 37 | CTCAGACCCAGTCTGGTGGT AAGCACTTGTACTCTAAC GCTTTGAACGAC |
| KLK7-2Q-R | SEQ ID NO: 38 | ACCAGACTGGGTCTGAGAGGTGATGATACCTCTAACACAC |
| SASPase-2Q-F | SEQ ID NO: 39 | CTCAGACCCAGTCT GGTGGT GTTTCTCAAGTTTACCCAATCGTTCAA GC |
| SASPase-2Q-R | SEQ ID NO: 40 | ACCAGACTGGGTCTGAGAGGTGATGATACCTCTAACACAC |
| Caspase-14-2Q-F | SEQ ID NO: 41 | CTCAGACCCAGTCTGGTGGT TGGGAACAC |
| Caspase-14-2Q-R | SEQ ID NO: 42 | ACCAGACTGGGTCTGAGAGGTGATGATACCTCTAACACAC |
| SNAP25 cleavage site 17-aa (187-203) | SEQ ID NO: 43 | SNKTRIDEANQRATKML |
| SNAP25 cleavage site 66-aa (141-206) | SEQ ID NO: 44 | ARENEMDENLEQVSGIIGNLRHMALDMGNEIDTQNRQIDRIME KADSNKTRIDEANQRATKMLGSG |
| CFP | SEQ ID NO: 45 | MSKGEELFTGVVPILVELDGDVNGHKFSVSGEGEGDATYGKLTL KFICTTGKLPVPWPTLVTTLTWGVQCFSRYPDHMKQHDFFKSA MPEGYVQERTIFFKDDGNYKTRAEVKFEGDTLVNRIELKGIDFK EDGNILGHKLEYNYISHNVYITADKQKNGIKANFKIRHNIEDGS VQLADHYQQNTPIGDGPVLLPDNHYLSTQSALSKDPNEKRDH MVLLEFVTAAGITHGMDELYK |
| YFP | SEQ ID NO: 46 | MSKGEELFTGVVPILVELDGDVNGHKFSVSGEGEGDATYGKLTL KFICTTGKLPVPWPTLVTTFGYGLQCFARYPDHMKRHDFFKSAM PEGYVQERTIFFKDDGNYKTRAEVKFEGDTLVNRIELKGIDFKE DGNILGHKLEYNYNSHNVYIMADKQKNGIKVNFKIRHNIEDGS VQLADHYQQNTPIGDGPVLLPDNHYLSYQSALSKDPNEKRDHM VLLEFVTAAGITHGMDELYK |
| **CFP-SNAP25/17aa-YFP** | SEQ ID NO: 47 | MSKGEELFTGVVPILVELDGDVNGHKFSVSGEGEGDATYGKLTL KFICTTGKLPVPWPTLVTTLTWGVQCFSRYPDHMKQHDFFKSA MPEGYVQERTIFFKDDGNYKTRAEVKFEGDTLVNRIELKGIDFK EDGNILGHKLEYNYISHNVYITADKQKNGIKANFKIRHNIEDGS VQLADHYQQNTPIGDGPVLLPDNHYLSTQSALSKDPNEKRDH MVLLEFVTAAGITHGMDELYKSNKTRIDEANQRATKMLMSKGE ELFTGVVPILVELDGDVNGHKFSVSGEGEGDATYGKLTLKFICTT GKLPVPWPTLVTTFGYGLQCFARYPDHMKRHDFFKSAMPEGYV QERTIFFKDDGNYKTRAEVKFEGDTLVNRIELKGIDFKEDGNILG HKLEYNYNSHNVYIMADKQKNGIKVNFKIRHNIEDGSVQLADH YQQNTPIGDGPVLLPDNHYLSYQSALSKDPNEKRDHMVLLEFV TAAGITHGMDELYK |
| **CFP-SNAP25/66aa-YFP** | SEQ ID NO: 48 | MSKGEELFTGVVPILVELDGDVNGHKFSVSGEGEGDATYGKLTL KFICTTGKLPVPWPTLVTTLTWGVQCFSRYPDHMKQHDFFKSA MPEGYVQERTIFFKDDGNYKTRAEVKFEGDTLVNRIELKGIDFK EDGNILGHKLEYNYISHNVYITADKQKNGIKANFKIRHNIEDGS VQLADHYQQNTPIGDGPVLLPDNHYLSTQSALSKDPNEKRDH MVLLEFVTAAGITHGMDELYKARENEMDENLEQVSGIIGNLRH MALDMGNEIDTQNRQIDRIMEKADSNKTRIDEANQRATKMLG SGMSKGEELFTGVVPILVELDGDVNGHKFSVSGEGEGDATYGKL TLKFICTTGKLPVPWPTLVTTFGYGLQCFARYPDHMKRHDFFKS AMPEGYVQERTIFFKDDGNYKTRAEVKFEGDTLVNRIELKGIDF KEDGNILGHKLEYNYNSHNVYIMADKQKNGIKVNFKIRHNIED GSVQLADHYQQNTPIGDGPVLLPDNHYLSYQSALSKDPNEKRD HMVLLEFVTAAGITHGMDELYK |
| SNAP25 | SEQ ID NO: 49 | MAEDADMRNELEEMQRRADQLADESLESTRRMLQLVEESKDA GIRTLVMLDEQGEQLDRVEEGMNHINQDMKEAEKNLKDLGKC CGLFICPCNKLKSSDAYKKAWGNNQDGVVASQPARVVDEREQ MAISGGFIRRVTNDARENEMDENLEQVSGIIGNLRHMALDMGN EIDTQNRQIDRIMEKADSNKTRIDEANQRATKMLGSG |
| First enzyme cleavage site (cleavable by Furin protease) | SEQ ID NO: 50 | RXXR |
| First enzyme cleavage site (cleavable by Furin protease) | SEQ ID NO: 51 | RRXX |

## Claims

1. A single-chain polypeptide, comprising: a first domain, an intermediate amino acid sequence region, and a second domain;
wherein
the intermediate amino acid sequence region is located between the first domain and the second domain;
the intermediate amino acid sequence region contains at least one first enzyme cleavage site; the first enzyme cleavage site is designed to be capable of being highly specifically cleaved by a first protease present at a specific site of the human body, and the single-chain polypeptide is converted into an activated form capable of inhibiting the release of a signaling substance upon cleavage of the first enzyme cleavage site by the first protease.

2. The single-chain polypeptide according to claim 1, wherein
the first domain has a molecular weight of approximately 50 kDa, the second domain has a molecular weight of approximately 100 kD, and the first domain possesses cleavage activity.

3. The single-chain polypeptide according to claim 1 or 2, wherein
the signaling substance is an intercellular signaling substance, preferably any one of a neurotransmitter, an endocrine hormone, a local chemical mediator, and a gaseous signaling molecule; more preferably, the signaling substance is acetylcholine.

4. The single-chain polypeptide according to any one of claims 1 to 3, wherein
the first domain is a non-toxic cellular protease or a fragment thereof capable of cleaving an exocytosis fusion protein of a target cell;
the second domain comprises a binding moiety and a transporting moiety;
the binding moiety is capable of binding to a receptor of the target cell and entering an endosome of the target cell via receptor-mediated endocytosis, and the transporting moiety is capable of transporting the first domain into a cytosol of the target cell;
preferably, the first enzyme cleavage site is located between the first domain and the transporting moiety; and/or the binding moiety is located at the carboxyl terminus with a molecular weight of approximately 50 kD, and the transporting moiety is located at the amino terminus with a molecular weight of approximately 50 kD;
more preferably, the exocytosis fusion protein is a neuronal SNARE protein, and/or the target binding site is a receptor on the presynaptic membrane of a neuron.

5. The single-chain polypeptide according to any one of claims 1 to 4, wherein
the first domain comprises at least a portion of a *Clostridium botulinum* neurotoxin L chain;
preferably, the portion of the *Clostridium botulinum* neurotoxin L chain is a neurotoxin L chain of one of *Clostridium botulinum* subtypes A, B, C, D, and E or is derived therefrom; and/or the first domain comprises an amino acid sequence having at least 35%, 50%, 60%, 70%, 75%, 80%, 85%, 90%, 99%, or 100% identity to SEQ ID NO: 10, or comprises an amino acid sequence having at least 35%, 50%, 60%, 70%, 75%, 80%, 85%, 90%, 99%, or 100% identity to any one of SEQ ID NOs: 11 to 16 with removal of a naturally occurring cleavage sequence; and/or
the second domain comprises at least a portion of a *Clostridium botulinum* neurotoxin H chain; preferably, the portion of the *Clostridium botulinum* neurotoxin H chain is a neurotoxin H chain of one of *Clostridium botulinum* (*C. Botulinum*) subtype A, *Clostridium botulinum* subtype B, *Clostridium botulinum* subtype C, *Clostridium botulinum* subtype D, *Clostridium botulinum* subtype E, *Clostridium botulinum* subtype F, *Clostridium botulinum* subtype G, *Clostridium baratii* (*C. Baratii*), and *Clostridium butyricum* (*C Butyricum*), or is derived therefrom; or the portion of the *Clostridium botulinum* neurotoxin H chain is a neurotoxin H chain of *Clostridium tetani* (*C. Tetani*) or is derived therefrom; and/or
the second domain comprises an amino acid sequence having at least 35%, 50%, 60%, 70%, 75%, 80%, 85%, 90%, 99%, or 100% identity to any one of SEQ ID NOs: 17 to 23.

6. The single-chain polypeptide according to any one of claims 1 to 7, wherein
the number of amino acids in the linker (L1) between the first domain and the intermediate amino acid sequence region is 0 to 20, 0 to 10, or 0 to 5; and/or
the number of amino acids in the linker (L2) between the intermediate amino acid sequence region and the second domain is 0 to 20, 0 to 10, or 0 to 5.

7. The single-chain polypeptide according to any one of claims 1 to 6, wherein
the first protease is not a protease present in the human digestive tract;
preferably, the specific site of the human body is human skin tissue, and/or at least one of the first proteases is selected from the group consisting of:
an epidermal-specific kallikrein-related peptidase (KLK) (*e.g.,* KLK5, KLK6, KLK7), a SASPase protease, a Caspase protease (*e.g*., Caspase-14), a Furin protease, and any one or more of their respective variants retaining proteolytic activity; and/or
the first enzyme cleavage site comprises an amino acid sequence having at least 50%, 60%, 85%, 90%, 99%, or 100% identity to any one of SEQ ID NOs: 1 to 4, SEQ ID NO: 50, and SEQ ID NO: 51;
wherein in SEQ ID NO: 50 and SEQ ID NO: 51, "X" in RXXR and RRXX is any amino acid; preferably, "XX" in RXXR is RK, TK, or TR; "XX" in RRXX is SV or KR.

8. The single-chain polypeptide according to any one of claims 1 to 7, wherein the single-chain polypeptide further comprises:
any one or more of a signal peptide for increased expression, a quantitative protein for quantification, and a tag protein;
further, one or more of the signal peptide, the quantitative protein, and the tag protein are respectively defined as follows:
the signal peptide comprises an amino acid sequence having at least 80%, 85%, 90%, 99%, or 100% identity to SEQ ID NO: 24;
the quantitative protein is a fluorescent protein;
the tag protein is one or more of a His tag, a GST tag, an MBP tag, and a Strep-tagII tag.

9. The single-chain polypeptide according to any one of claims 1 to 8, wherein the single-chain polypeptide further comprises:
two second enzyme cleavage sites located at the N-terminus of the first domain and the C-terminus of the second domain, respectively, and a second protease that cleaves the second enzyme cleavage site is incapable of cleaving the first enzyme cleavage site.

10. The single-chain polypeptide according to any one of claims 1 to 9, wherein
the first enzyme cleavage site and/or the second enzyme cleavage site are typically not cleaved by proteases encountered during expression of the single-chain polypeptide within a host cell or during synthesis of the single-chain polypeptide in an *in vitro* synthesis system involving a cell extract derived from the host cell.

11. The single-chain polypeptide according to claim 9 or 10, wherein
the second enzyme cleavage site is designed to be highly specific for the second protease;
preferably, the second protease is selected from a non-human enterokinase, a tobacco etch virus protease, a protease derived from *Bacillus subtilis,* a protease derived from *Bacillus amyloliquefaciens,* a protease derived from rhinovirus, papain, a homolog of papain of an insect, or a homolog of papain of a crustacean; and/or
the second enzyme cleavage site comprises an amino acid sequence having at least 85%, 90%, 99%, or 100% identity to any one of SEQ ID NOs: 5 to 9.

12. The single-chain polypeptide according to any one of claims 1 to 11, wherein
the intermediate amino acid sequence region further comprises XTXS, wherein the X is an amino acid other than K; preferably, the X is Q; and/or
the structure of the intermediate amino acid sequence region from the N-terminus to the C-terminus is:
XTXS-linker (L3)-at least one first enzyme cleavage site; further, the number of amino acids in L3 is 0 to 20, 0 to 10, or 0 to 5; further, there is one first enzyme cleavage site.

13. The single-chain polypeptide according to any one of claims 1 to 12, wherein
when the second domain comprises an amino acid sequence having at least 80%, 85%, 90%, 95%, 99%, or 100% identity to SEQ ID NO: 17, the lysine at the position corresponding to position 423 of SEQ ID NO: 17 in the amino acid sequence of the second domain is mutated to a non-lysine residue.

14. The single-chain polypeptide according to any one of claims 1 to 13, wherein
the first domain, the intermediate amino acid sequence region, and the second domain are connected from the N-terminus to the C-terminus; and/or
when there are multiple first enzyme cleavage sites, the multiple first enzyme cleavage sites are sequentially connected from the N-terminus to the C-terminus, wherein all of the first enzyme cleavage sites are identical or different from one another, and the number of amino acids in the linker (L4) between the multiple enzyme cleavage sites is 0 to 20, 0 to 10, or 0 to 5.

15. Use of the single-chain polypeptide according to any one of claims 1 to 14, wherein
the use comprises any one or more of following: skin care, plastic surgery, body contouring, and therapy by inducing muscle relaxation or relief of muscle spasm.

16. Use of the single-chain polypeptide according to any one of claims 1 to 14 in the manufacture of a product or medicament for a specific purpose, wherein
the specific purpose comprises any one or more of following: skin care, plastic surgery, body contouring, and therapy by inducing muscle relaxation or relief of muscle spasm.

17. A product or medicament of a single-chain polypeptide, comprising:
the single-chain polypeptide according to any one of claims 1 to 14;
preferably, the product or medicament has a use comprising any one or more of following: skin care, plastic surgery, body contouring, and therapy by inducing muscle relaxation or relief of muscle spasm;
more preferably, the product or medicament is applied to a specific site of the human body by means of topical application, spraying, external application, or injection.

18. A nucleic acid, comprising:
a nucleotide sequence encoding the single-chain polypeptide according to any one of claims 1 to 14.

19. A vector, comprising:
the nucleic acid according to claim 18.

20. A host cell, integrated with the nucleic acid according to claim 18 and/or comprising the vector according to claim 19.

21. The host cell according to claim 20, wherein
the host cell is derived from a prokaryotic cell or a eukaryotic cell; further, the cell is selected from the following groups: one or more of an *Escherichia coli* cell, a human-derived cell, a Chinese hamster ovary cell, an insect cell, a wheat germ cell, a rabbit reticulocyte, and a yeast cell, or a combination thereof.

22. The host cell according to claim 21, wherein
the host cell is derived from a yeast cell; further, the yeast cell is selected from one or more of *Saccharomyces cerevisiae* and *Kluyveromyces,* or a combination thereof; in another preferred example, the *Kluyveromyces* is selected from one or more of *Kluyveromyces lactis, Kluyveromyces marxianus,* and *Kluyveromyces dobzhanskii,* or a combination thereof.

23. An *in vitro* cell-free synthesis system, wherein
an mRNA or DNA encoding the single-chain polypeptide according to any one of claims 1 to 14 is used as a template to synthesize the single-chain polypeptide.

24. The *in vitro* cell-free synthesis system according to claim 23, wherein the *in vitro* cell-free synthesis system comprises any one or more of the following:
(1) an mRNA or DNA template encoding the single-chain polypeptide;
(2) a cell extract, wherein the cell extract is a yeast cell extract; further, the cell extract is derived from any one or more of *Saccharomyces cerevisiae, Pichia pastoris,* and *Kluyveromyces,* or a combination thereof; preferably, the yeast cell extract is derived from *Kluyveromyces,* more preferably *Kluyveromyces lactis*;
(3) any one or more of the following components:
an amino acid mixture, dNTP, RNA polymerase, DNA polymerase, an energy supply system, polyethylene glycol, and an aqueous solvent.

25. A preparation method for the single-chain polypeptide according to any one of claims 1 to 14, wherein
an mRNA or DNA encoding the single-chain polypeptide according to any one of claims 1 to 14 is used as a template to perform an *in vitro* synthesis reaction to obtain the single-chain polypeptide;
preferably, the *in vitro* synthesis reaction is performed in a reaction system comprising the *in vitro* cell-free synthesis system according to claim 23 or 24;
further, the DNA template and other components involved in the *in vitro* synthesis reaction have a volume ratio of 1:10 to 1:50, or 1:20 to 1:40, or 1:25 to 1:35, or 1:30.

26. The preparation method according to claim 25, wherein
the *in vitro* cell-free synthesis system comprises a yeast cell extract, an amino acid mixture, dNTP, RNA polymerase, DNA polymerase, an energy supply system, polyethylene glycol, and an aqueous solvent;
further:
the yeast cell extract is derived from any one or more of *Saccharomyces cerevisiae, Pichia pastoris,* and *Kluyveromyces,* or a combination thereof; preferably, the yeast cell extract is derived from *Kluyveromyces,* more preferably *Kluyveromyces lactis.*

27. The preparation method according to claim 26, wherein
the yeast cell extract accounts for 50 to 80% of the total volume of the *in vitro* cell-free protein synthesis system.

28. The preparation method according to any one of claims 25 to 27, wherein the preparation method further comprises:
digesting the obtained single-chain polypeptide comprising the second enzyme cleavage site with the second protease to obtain a single-chain polypeptide comprising only the first domain, the intermediate amino acid sequence region, and the second domain.

29. Use of the nucleic acid according to claim 18, the vector according to claim 19, the host cell according to any one of claims 20 to 22, and the *in vitro* cell-free synthesis system according to claim 23 or 24 in the manufacture of the single-chain polypeptide according to any one of claims 1 to 13.
